(19) Europäisches Patentamt
European Patent Office
Office européen des brevets

(11) Numéro de publication: **0 205 362 B1**

(12) # FASCICULE DE BREVET EUROPEEN

(45) Date de publication du fascicule du brevet: **20.12.89**

(51) Int. Cl.⁴: **C 07 D 215/54,** C 07 D 215/42, C 07 D 401/12, A 61 K 31/47

(21) Numéro de dépôt: **86400973.3**

(22) Date de dépôt: **06.05.86**

(54) **Dérivés quinolylglycinamides, leur procédé de préparation et leur application thérapeutique en tant que psychotropes.**

(30) Priorité: **06.05.85 FR 8507247**

(43) Date de publication de la demande: **17.12.86 Bulletin 86/51**

(45) Mention de la délivrance du brevet: **20.12.89 Bulletin 89/51**

(84) Etats contractants désignés: **AT BE CH DE FR GB IT LI LU NL SE**

(56) Documents cités:
**GB-A-1 177 548**
**US-A-2 456 911**
**US-A-3 470 186**
**US-A-3 769 410**

(73) Titulaire: **SANOFI, 40, Avenue George V, F-75008 Paris (FR)**

(72) Inventeur: **Vernieres, Jean-Claude, rue Sabatier Garat, F-31600 Muret (FR)**
Inventeur: **Mendes, Etienne, 2 place Jeanne d'Arc, F-31000 Toulouse (FR)**
Inventeur: **Keane, Peter, 10 avenue Winston Churchill, F-31100 Toulouse (FR)**
Inventeur: **Simiand, Jacques, 136 chemin de Lacombe, F-31600 Muret (FR)**
Inventeur: **Morre, Michel, 11 rue Sainte-Odile, F-31100 Toulouse (FR)**

(74) Mandataire: **Bressand, Georges et al, c/o CABINET LAVOIX 2 Place d'Estienne d'Orves, F-75441 Paris Cédex 09 (FR)**

EP 0 205 362 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention est relative à des nouveaux dérivés quinolylglycinamides, à leur procédé de préparation et à leur application thérapeutique en tant que psychotropes.

US-A-3 769 410 décrit des quinolyl-4-glycinamides d'activité antalgique et anti-inflammatoire.

US-A-2 456 911 décrit des quinolyl-4-glycinamides à activité antispasmodique et anesthésique locale.

Les composés de l'invention répondent à la formule générale suivante:

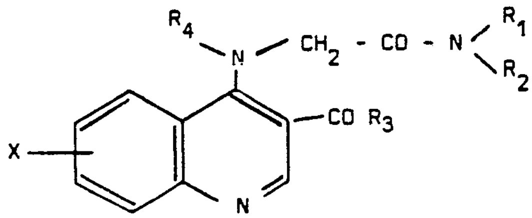

(I)

dans laquelle

$R_1$ et $R_2$ représentent chacun indépendamment l'un de l'autre un hydrogène ou un radical alcoyle inférieur ou bien, lorsque $R_1$ est un radicale alcoyle, $R_2$ peut représenter un radical cycloalcoyle, un radical phényle ou benzyle éventuellement substitués par un halogène ou un alcoyle inférieur, ou bien encore $R_1$ et $R_2$ peuvent former avec l'atome d'azote auquel ils sont rattachés un hétérocycle pouvant comporter un second hétéroatome tel que l'oxygène, le soufre ou l'azote;

$R_3$ représente un hydrogène, un hydroxyle OH, un groupe alcoyle ou alcoxy inférieurs, phénoxy ou benzyloxy éventuellement substitués, par un halogène ou un alcoyle inférieur, ou bien $R_3$ représente un groupement -NH-$R_5$ dans lequel $R_5$ est un hydrogène, un radical alcoyle inférieur ou phényle;

$R_4$ représente un hydrogène ou un radical alcoyle inférieur;

X représente un atome d'hydrogène ou un halogène, un radical alcoyle inférieur, cycloalcoyle, alcoxy, nitro, trifluorométhyle ou méthylthio.

L'invention concerne aussi les sels d'addition avec les acides minéraux ou organiques pharmaceutiquement acceptables et éventuellement, lorsque $R_3$ représente OH, les sels avec les bases alcalines (NaOH, KOH) ou organiques (pipérazine, lysine, morpholine), pharmaceutiquement acceptables.

Par radical alcoyle ou alcoxy inférieur, on entend un radical hydrocarboné en $C_1$-$C_4$, linéaire ou ramifié, saturé ou non; par hétérocycle, on entend notamment un radical pyrrolidino, pipéridino, homopipéridino (héxaméthylèneimino) ou morpholino pouvant porter un substituant alcoyle en $C_1$-$C_4$.

L'invention a également pour objet un procédé de préparation des composés de formule (I) caratérisé en ce que l'on fait réagir un dérivé de la glycine de formule

$$R_4\text{-HN-}CH_2\text{-CO-N}\langle{}^{R_1}_{R_2} \qquad \text{(II)}$$

dans laquelle $R_1$, $R_2$ et $R_4$ sont tels que définis ci-dessus, avec une chloro-4 quinoléine de formule

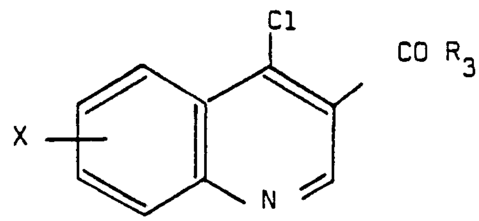

(III)

dans laquelle X et $R_3$ ont les valeurs définies précédemment.

La réaction de condensation est effectuée par chauffage des 2 réactifs à une température comprise entre 60 °C et 130 °C en présence d'une base organique telle que la triéthylamine, ou minérale telle que le carbonate de sodium. Cette réaction est avantageusement réalisée dans un solvant organique tel que le toluène, l'alcool éthylique ou isopropylique.

Les composés de formule (I) dans lesquels $R_3$ représente OH peuvent aussi être obtenus par saponification dans l'hydroxyde de sodium ou de potassium des esters de formule (I) dans lesquels $R_3$ est un groupement alcoxy, ou par hydrogénation en présence de catalyseurs, des esters de formule (I) dans lesquels $R_3$ est un groupement benzyloxy.

Les dérivés de la glycine de formule (II), qui sont des composés connus, ont été préparés par 3 méthodes:

a) lorsque $R_4$ est l'hydrogène, on obtient les dérivés sous forme de chlorhydrate (R.D. HAWORTH et al., J. Chem. Soc., 1952, 2972-2980) selon le schéma suivant:

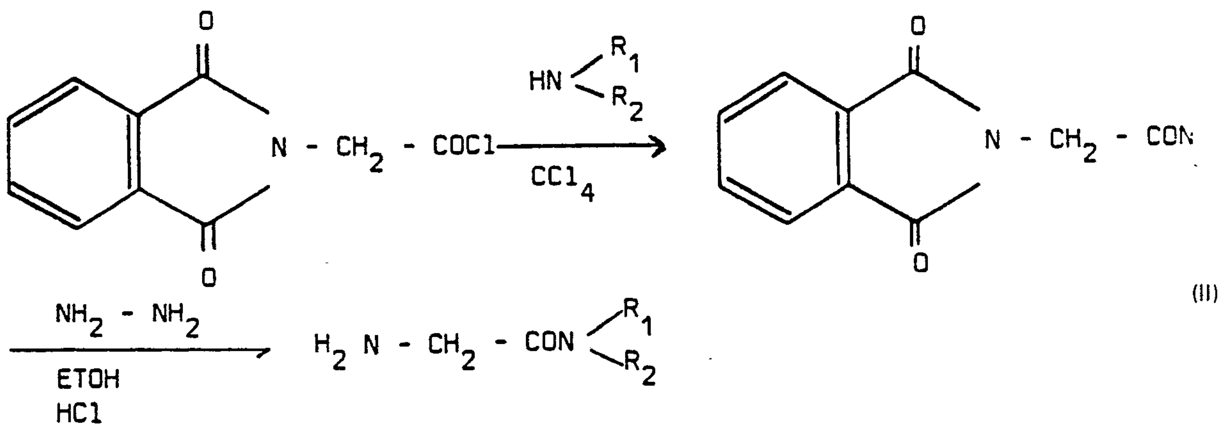


(II)

b) lorsque $R_4$ est un groupe méthyle, les dérivés de formule (II) sont préparés à partir d'esters de la sarcosine de fomule

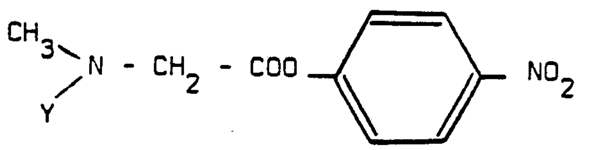

Y étant un groupement protecteur représentant soit un groupe benzyloxycarbonyl de formule

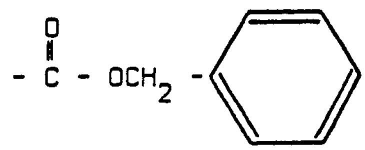

(A.B. MAUGER et R. WADE, J. Chem. Soc., 1965, 3126-3132)

soit un groupe tert-butyloxycarbonyl,

O
||
$-C-OC(CH_3)_3$

(Chem. Abstracts, 1970, 73, 56417 r).

En traitant ces esters par des amines de formule

$R_1$
H-N<
$R_2$

on obtient des amides de formule

$CH_3$ $R_1$
$>N-CH_2-CON<$
Y $R_2$

puis après élimination du groupe protecteur Y le dérivé de formule

$CH_3$ $R_1$
$>N-CH_2-CON<$
H $R_2$

c) en traitant du chlorure de chloroacétyle d'abord par une amine de formule

$R_1$
HN< puis par une amine du type $H_2N-R_4$
$R_2$

$R_1$
HN<
$R_2$ $R_1$
$Cl-CH_2-COCl$ ⟶ $Cl-CH_2-CON<$
$R_2$

$H_2N-R_4$ $R_4$ $R_1$
⟶ $>N-CH_2-CON<$
H $R_2$

(G.B.M. BETHOLO et J.F. CAVALLA, Gazz. Chim. Ital., 1954, 84 896-907).

Les dérivés de formule (III), les chloro-4 quinoléines sont des composés connus. Ainsi, C.E. KASLOW et W.R. CLARK (J. Org. Chem., 1953, 18, 55-58), J.W. HANIFIN et al. (J. Med. Chem., 1969, 12, 1096-1097) et E.H. ERICKSON et al. (J. Med. Chem. 1979, 22, 816-823) ont décrit les chloro-4 éthoxycarbonyl-3 quinoléines; C.C. PRICE et al. (J.A.C.S., 1946, 68, 1251-1252) et C.J. OHNMACHT et al. (J. Med. Chem., 1971, 14, 17-24) ont décrit des chlorocarbonyl-3 chloro-4 quinoléines de formule

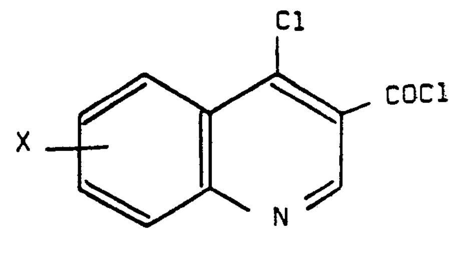

qui permettent par des réactions classiques avec des alcools ou des amines d'obtenir le dérivé de formule (III) recherché.

Les exemples non limitatifs suivant illustrent l'invention:

*Exemple 1*

[(éthoxycarbonyl-3 chloro-6) quinolyl-4] amino-2 N,N-diéthylactétamide; dérivé N° 1.
($R_1 = R_2 = C_2H_5$; $R_3 = OC_2H_5$; $R_4 = H$; $X = Cl$)

Un mélange de dichloro-4,6 quinoline carboxylate d'éthyle-3 (4 g, 0,016 mole), de chlorhydrate d'amino-2 N,N-diéthylacétamide (2,9 g, 0,0176 mole) préparé selon R.D. HAWORTH et al., (J. Chem. Soc., 1952, 2972-80) et de triéthylamine (4,9 ml, 0,0352 mole) dans l'éthanol (40 ml) est porté 5 heures au reflux sous azote. Après évaporation du solvant, les cristaux obtenus sont solubilisés dans le dichlorométhane. On lave à l'eau, sèche sur sulfate de sodium et évapore le solvant. Le produit attendu est recristallisé dans un mélange éther isopropylique-acétate d'éthyle (4-6): cristaux incolores, F = 101 °C (rendement 67%).

Analyse: $C_{18}H_{22}N_3ClO_3$

| | | | |
|---|---|---|---|
| Calculé: | C 59,42 | H 6,09 | N 11,55 |
| Trouvé: | C 59,31 | H 6,05 | N 11,63 |

Le maléate de ce composé a été préparé. On obtient des cristaux incolores.
F = 147°C (rendement 50%)

Analyse: $C_{22}H_{26}ClN_3O_7$

| | | | |
|---|---|---|---|
| Calculé: | C 55,06 | H 5,46 | N 8,76 |
| Trouvé: | C 55,09 | H 5,60 | N 8,56 |

*Exemple 2*

[(éthoxycarbonyl-3 méthyl-6) quinolyl-4] amino-2 N,N-diéthylacétamide, chlorhydrate; dérivé N° 2.
($R_1 = R_2 = C_2H_5$; $R_3 = OC_2H_5$; $R_4 = H$; $X = CH_3$)

2,49 g (0,01 mole) de méthyl-6 chloro-4 quinoline carboxylate d'éthyl-3, 1,83 g (0,011 mole) d'amino-2 N,N-diéthylacétamide, 3,1 ml (0,022 mole) de triéthylamine et 20 ml d'éthanol absolu sont portés quinze heures au reflux. L'éthanol éliminé, le résidu est repris par 10 ml d'eau puis après extraction au dichlorométhane, la phase organique est lavée par le minimum d'eau, séchée et concentrée. Le chlorhydrate est recristallisé dans l'isopropanol: cristaux incolores, F > 260°C (rendement 50%).

Analyse: $C_{19}$ $H_{25}N_3O_3HCl$

| | | | |
|---|---|---|---|
| Calculé: | C 60,07 | H 6,90 | N 11,06 |
| Trouvé: | C 60,27 | H 6,87 | N 10,88 |

En suivant le mode opératoire décrit dans l'exemple 1, on a préparé successivement à partir des chloro-4 quinolines diversement substitués en position 6 et le chlorhydrate d'amino-2 N,N-diéthylacétamide, les composés des exemples de 3 à 11 suivants:

*Exemple 3*

[(éthoxycarbonyl-3 méthoxy-6) quinolyl-4] amino-2 N,N-diéthylacétamide; dérivé N° 3.
($R_1=R_2=C_2H_5$; $R_3=OC_2H_5$; $R_4=H$; $X=OCH_3$)
F = 163°C (rendement 68%)

Analyse: $C_{19}H_{25}N_3O_4$

| | | | |
|---|---|---|---|
| Calculé: | C 63,49 | H 7,01 | N 11,69 |
| Trouvé: | C 63,46 | H 6,99 | N 11,61 |

*Exemple 4*

[(éthoxycarbonyl-3 trifluorométhyl-6) quinolyl-4] amino-2 N,N-diéthylacétamide; derivé N° 4.
($R_1=R_2=C_2H_5$; $R_3=OC_2H_5$; $R_4=H$; $X=CF_3$)
F = 136°C (rendement 50%)

Analyse: $C_{19}H_{22}F_3O_3$

| | | | |
|---|---|---|---|
| Calculé: | C 57,43 | H 5,58 | N 10,57 |
| Trouvé: | C 57,31 | H 5,66 | N 10,76 |

*Example 5*

[(éthoxycarbonyl-3 nitro-6) quinolyl-4] amino-2 N,N-diéthylacétamide; dérivé N° 5.
($R_1=R_2=C_2H_5$; $R_3=OC_2H_5$; $R_4=H$, $X=NO_2$)
F = 170°C (rendement 70%)

Analyse: $C_{18}H_{22}N_4O_5$

| | | | |
|---|---|---|---|
| Calculé: | C 57,74 | H 5,32 | N 14,97 |
| Trouvé: | C 57,53 | H 5,70 | N 14,86 |

*Exemple 6*

[(éthoxycarbonyl-3 butyl-6) quinolyl-4] amino-2 N,N-diéthylacétamide; dérivé N° 6.
($R_1=R_2=C_2H_5$; $R_3=OC_2H_5$; $R_4=H$; $X=C_4H_9$
F = 128°C (rendement 70%)

Analyse: $C_{22}H_{31}N_3O_3$

| | | | |
|---|---|---|---|
| Calculé: | C 68,54 | H 8,10 | N 10,90 |
| Trouvé: | C 68,51 | H 8,32 | N 10,91 |

*Exemple 7*

[(éthoxycarbonyl-3 fluoro-6) quinolyl-4] amino-2 N,N-diéthylacétamide; dérivé N° 7.
($R_1=R_2=C_2H_5$; $R_3=OC_2H_5$; $R_4=H$; $X=F$)
F = 126°C (rendement 60%)

Analyse: $C_{18}H_{22}FN_3O_3$

| | | | |
|---|---|---|---|
| Calculé: | C 62,24 | H 6,38 | N 12,10 |
| Trouvé: | C 62,00 | H 6,30 | N 12,03 |

*Exemple 8*

[(éthoxycarbonyl-3 éthoxy-6) quinolyl-4] amino-2 N,N-diéthylacétamide; dérivé N° 8.
($R_1=R_2=C_2H_5$; $R_3=OC_2H_5$; $R_4=H$; $X=OC_2H_5$)
F = 184°C (rendement 55%)

Analyse: $C_{20}H_{27}N_3O_4$

| | | | |
|---|---|---|---|
| Calculé: | C 64,32 | H 7,29 | N 11,25 |
| Trouvé: | C 64,16 | H 7,23 | N 11,13 |

*Exemple 9*

[(éthoxycarbonyl-3 cyclohexyl-6) quinolyl-4] amino-2 N,N-diéthylacétamide; dérivé N° 9.
($R_1=R_2=C_2H_5$; $R_3=OC_2H_5$; $R_4=H$; $X=$cyclohexyl)
F = 130°C (rendement 60%)

Analyse: $C_{24}H_{33}N_3O_3$

| | | | |
|---|---|---|---|
| Calculé: | C 70,04 | H 8,08 | N 10,21 |
| Trouvé: | C 70,26 | H 8,32 | N 9,96 |

*Exemple 10*

[(éthoxycarbonyl-3 quinolyl-4)] amino-2 N,N-diéthylacétamide; dérivé N° 10.
($R_1=R_2=C_2H_5$; $R_3=OC_2H_5$; $R_4=H$; $X=H$)
F = 120°C (rendement 70%)

Analyse: $C_{18}$ $H_{23}$ $N_3O_3$

| | | | |
|---|---|---|---|
| Calculé: | C 65,63 | H 7,04 | N 12,76 |
| Trouvé: | C 65,74 | H 7,17 | N 12,77 |

*Exemple 11*

[(éthoxycarbonyl-3 butoxy-6) quinolyl-4] amino-2 N,N-diéthylacétamide; dérivé N° 11.
($R_1=R_2=C_2H_5$; $R_3=OC_2H_5$; $R_4=H$; $X=OC_4H_9$)
F = 163°C (rendement 50%)

Analyse: $C_{22}H_{31}N_3O_4$

| | | | |
|---|---|---|---|
| Calculé: | C 65,81 | H 7,78 | N 10,47 |
| Trouvé: | C 65,88 | H 7,59 | N 10,50 |

*Exemple 12*

[(éthoxycarbonyl-3 chloro-6) quinolyl-4] amino-2 N,N-dipropylacétamide; dérivé N° 12.
($R_1=R_2=C_3H_7$; $R_3=OC_2H_5$; $R_4=H$; $X=Cl$)

Un mélange de dichloro-4,6 quinoline carboxylate d'éthyle-3 (2,7 g, 0,01 mole), de chlorhydrate d'amino-2 N,N-dipropylacétamide (2,14 g, 0,011 mole) et de triéthylamine (2,22 g, 0,022 mole) dans l'éthanol (60 ml) est porté au reflux 4 heures. Après concentration du solvant, lavage à l'eau, extraction au dichlorométhane, séchage sur $Na_2SO_4$ et évaporation du solvant, on recristallise dans un mélange cyclohexane-acétate d'éthyle (90-10) le produit attendu: cristaux beiges, F = 116°C (rendement 60%).

Analyse: $C_{20}H_{26}ClN_3O_3$

| | | | |
|---|---|---|---|
| Calculé: | C 61,30 | H 6,69 | N 10,72 |
| Trouvé: | C 61,10 | H 6,40 | N 10,86 |

Le phosphate et le sulfate de ce composé ont été préparés. A titre d'exemple, on a obtenu le sulfate acide hydraté de l'exemple 12, cristaux incolores, F = 150°C.

Analyse: $C_{20}H_{28}ClN_3O_7S$, 1/2 $H_2O$

| | | | |
|---|---|---|---|
| Calculé: | C 48,13 | H 5,85 | N 8,41 |
| Trouvé: | C 47,90 | H 5,56 | N 8,36 |

En suivant le mode opératoire décrit dans l'exemple 12, on a préparé, successivement à partir du chlorhydrate d'amino-2 N,N-dipropylacétamide, les composés des exemples 13 à 20 suivants:

*Exemple 13*

[(éthoxycarbonyl-3 méthyl-6) quinolyl-4] amino-2 N,N-dipropylacétamide chlorhydrate; dérivé N° 13.
($R_1 = R_2 = C_3H_7$; $R_3 = OC_2H_5$; $R_4 = H$; $X = CH_3$)
F = 259°C (rendement 40%)

Analyse: $C_{21}H_{29}N_3O_3HCl$

| | | | |
|---|---|---|---|
| Calculé: | C 61,83 | H 7,41 | N 10,30 |
| Trouvé: | C 61,74 | H 7,24 | N 10,20 |

*Exemple 14*

[(éthoxycarbonyl-3 méthoxy-6) quinolyl-4] amino-2 N,N-dipropylacétamide; dérivé N° 14.
($R_1 = R_2 = C_3H_7$; $R_3 = OC_2H_5$; $R_4 = H$; $X = OCH_3$]
F = 163°C (rendement 70%)

Analyse: $C_{21}H_{29}N_3O_4$

| | | | |
|---|---|---|---|
| Calculé: | C 65,09 | H 7,54 | N 10,84 |
| Trouvé: | C 65,11 | H 7,65 | N 10,82 |

*Exemple 15*

[(éthoxycarbonyl-3 quinolyl-4)] amino-2 N,N-dipropylacétamide; dérivé N° 15.
($R_1 = R_2 = C_3H_7$; $R_3 = OC_2H_5$; $R_4 = H$; $X = H$)
Fusion: 120°C (rendement 60%)

Analyse: $C_{20}H_{27}N_3O_3$

| | | | |
|---|---|---|---|
| Calculé: | C 67,20 | H 7,61 | N 11,76 |
| Trouvé: | C 67,31 | H 7,76 | N 11,81 |

*Exemple 16*

[(éthoxycarbonyl-3 éthoxy-6) quinolyl-4] amino-2 N,N-dipropylacétamide; dérivé N° 16.
($R_1 = R_2 = C_3H_7$; $R_3 = OC_2H_5$; $R_4 = H$, $X = OC_2H_5$)
F = 176°C (rendement 55%)

Analyse: $C_{22}H_{31}N_3O_4$

| | | | |
|---|---|---|---|
| Calculé: | C 65,81 | H 7,78 | N 10,47 |
| Trouvé: | C 65,73 | H 7,94 | N 10,26 |

*Exemple 17*

[(éthoxycarbonyl-3 bromo-6) quinolyl-4] amino-2 N,N-dipropylacétamide; dérivé N° 17.
($R_1 = R_2 = C_3H_7$; $R_3 = OC_2H_5$; $R_4 = H$, $X = Br$)
F = 126°C (rendement 60%)

Analyse: $C_{20}H_{26}BrN_3O_3$

| | | | |
|---|---|---|---|
| Calculé: | C 55,05 | H 6,01 | N 9,63 |
| Trouvé: | C 55,17 | H 5,97 | N 9,66 |

*Exemple 18*

[(éthoxycarbonyl-3 nitro-6) quinolyl-4] amino-2 N,N-dipropylacétamide; dérivé N° 18.
($R_1 = R_2 = C_3H_7$; $R_3 = OC_2H_5$; $R_4 = H$; $X = NO_2$)
F = 160°C (rendement 50%)

Analyse: $C_{20}H_{26}N_4O_5$

| | | | |
|---|---|---|---|
| Calculé: | C 64,86 | H 5,68 | N 9,86 |
| Trouvé: | C 64,78 | H 5,63 | N 9,75 |

*Exemple 19*

[(éthoxycarbonyl-3 trifluorométhyl-6) quinolyl-4] amino-2 N,N-dipropylacétamide; dérivé N° 19.
($R_1 = R_2 = C_3H_7$; $R_3 = OC_2H_5$; $R_4 = H$, $X = CF_3$)
F = 144°C (rendement 60%)

Analyse: $C_{21}H_{26}F_3N_3O_3$

| | | | |
|---|---|---|---|
| Calculé: | C 59,28 | H 6,16 | N 9,87 |
| Trouvé: | C 58,98 | H 5,96 | N 9,67 |

*Exemple 20*

[(éthoxycarbonyl-3 méthylthio-6) quinolyl-4] amino--2 N,N-dipropylacétamide; dérivé N° 20.
($R_1 = R_2 = C_3H_7$; $R_3 = OC_2H_5$; $R_4 = H$; $X = SCH_3$)
F = 162°C (rendement 72%)

Analyse: $C_{21}H_{29}N_3O_3S$

| | | | |
|---|---|---|---|
| Calculé: | C 62,50 | H 7,24 | N 10,41 |
| Trouvé: | C 62,36 | H 7,47 | N 10,27 |

En suivant le mode opératoire décrit dans l'exemple 1, on a préparé, à partir des chlorhydrates d'amino-2 acétamide diversement substitués, les composés des exemples 21 à 42 suivants:

*Exemple 21*

[(éthoxycarbonyl-3 chloro-6) quinolyl-4] amino-2 N,N-diisobutylacétamide; dérivé N° 21.
($R_1 = R_2 = CH_2\text{-}CH(CH_3)_2$; $R_3 = OC_2H_5$; $R_4 = H$; $X = Cl$)
F = 136°C (rendement 50%)

Analyse: $C_{22}H_{30}ClN_3O_3$

| | | | |
|---|---|---|---|
| Calculé: | C 62,92 | H 7,20 | N 10,00 |
| Trouvé: | C 62,72 | H 7,02 | N 9,93 |

*Exemple 22*

[(éthoxycarbonyl-3 chloro-6) quinolyl-4] amino-2 N-méthyl N-phényl acétamide; dérivé N° 22.
($R_1 = CH_3$; $R_2 = C_6H_5$; $R_3 = OC_2H_5$; $R_4 = H$; $X = Cl$)
Fusion 178°C (rendement 47%)

Analyse: $C_{21}H_{20}ClN_3O_3$

| | | | |
|---|---|---|---|
| Calculé: | C 63,40 | H 5,07 | N 10,56 |
| Trouvé: | C 63,38 | H 5,14 | N 10,59 |

*Exemple 23*

[(éthoxycarbonyl-3 chloro-6) quinolyl-4] amino-2 N-méthyl N-cyclohexyl acétamide; dérivé N° 23.
($R_1 = CH_3$; $R_2 = C_6H_{11}$; $R_3 = OC_2H_5$; $R_4 = H$; $X = Cl$)
Fusion 140°C (rendement 25%)

Analyse: $C_{21}H_{26}ClN_3O_3$

| | | | |
|---|---|---|---|
| Calculé: | C 62,45 | H 6,49 | N 10,40 |
| Trouvé: | C 61,91 | H 6,60 | N 10,18 |

*Exemple 24*

[(éthoxycarbonyl-3 chloro-6) quinolyl-4] amino-2 N-méthyl N-chloro-4 phényl acétamide; dérivé N° 24.
($R_1 = CH_3$; $R_2 = C_6H_5p\text{-}Cl$; $R_3 = OC_2H_5$; $R_4 = H$; $X = Cl$)
Fusion 196°C (rendement 30%)

Analyse: $C_{21}H_{19}Cl_2N_3O_3$

| | | | |
|---|---|---|---|
| Calculé: | C 58,35 | H 4,43 | N 9,72 |
| Trouvé: | C 58,20 | H 4,29 | N 9,66 |

*Exemple 25*

{[(éthoxycarbonyl-3 chloro-6 quinolyl-4) amino]-2 acétyl}-1-pipéridine; dérivé N° 25.

(N$<^{R_1}_{R_2}$ = 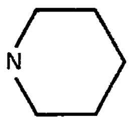 ; $R_3 = OC_2H_5$; $R_4 = H$; $X = Cl$)

Fusion = 148°C (rendement 37%)

Analyse: $C_{19}H_{22}ClN_3O_3$

| | | | |
|---|---|---|---|
| Calculé: | C 60,72 | H 5,90 | N 11,18 |
| Trouvé: | C 60,84 | H 5,69 | N 11,43 |

Le sel bis-phosphate de ce composé a été préparé.
Fusion = 185°C (rendement 45% à partir de la base)

*Exemple 26*

{[(éthoxycarbonyl-3 chloro-6 quinolyl-4) amino]-2 acétyl}-1-pyrrolidine; dérivé N° 26.

(N$<^{R_1}_{R_2}$ = 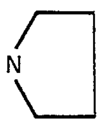 ; $R_3 = OC_2H_5$; $R_4 = H$; $X = Cl$)

Fusion = 170°C (rendement 60%)

Analyse: $C_{18}H_{20}ClN_3O_3$

| | | | |
|---|---|---|---|
| Calculé: | C 59,75 | H 5,57 | N 11,61 |
| Trouvé: | C 59,91 | H 5,42 | N 11,85 |

*Exemple 27*

{[(éthoxycarbonyl-3 chloro-6 quinolyl-4) amino] -2 acétyl}-1-homopipéridine; dérivé N° 27.

(N$<^{R_1}_{R_2}$ = 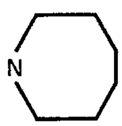 ; $R_3 = OC_2H_5$; $R_4 = H$; $X = Cl$)

Fusion: 129°C (rendement 37%)

Analyse: $C_{20}H_{24}ClN_3O_3$

| | | | |
|---|---|---|---|
| Calculé: | C 61,61 | H 6,20 | N 10,78 |
| Trouvé: | C 61,66 | H 6,29 | N 10,80 |

*Exemple 28*

{[(éthoxycarbonyl-3 chloro-6 quinolyl-4) amino] -2 acétyl}-1-morpholine; dérivé N° 28.

(N$<^{R_1}_{R_2}$ = 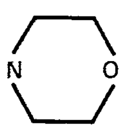 ; $R_3 = OC_2H_5$; $R_4 = H$; $X = Cl$)

Fusion: 171°C (rendement 20%)

Analyse: $C_{18}H_{20}ClN_3O_4$

| | | | |
|---|---|---|---|
| Calculé: | C 57,22 | H 5,34 | N 11,12 |
| Trouvé: | C 57,23 | H 5,12 | N 11,13 |

*Exemple 29*

[(éthoxycarbonyl-3 chloro-6) quinolyl-4] amino-2 N,N-diméthylacétamide; dérivé N° 29.
($R_1 = R_2 = CH_3$; $R_3 = OC_2H_5$; $R_4 = H$; $X = Cl$)
Fusion: 169°C (rendement 33%)

Analyse: $C_{16}H_{18}ClN_3O_3$

| | | | |
|---|---|---|---|
| Calculé: | C 57,23 | H 5,40 | N 12,51 |
| Trouvé: | C 57,51 | H 5,38 | N 12,37 |

*Exemple 30*

[(éthoxycarbonyl-3 chloro-6) quinolyl-4] amino-2 acétamide; dérivé N° 30.
($NR_1R_2 = NH_2$; $R_3 = OC_2H_5$; $R_4 = H$; $X = Cl$)
Fusion = 231°C (rendement 60%)

Analyse: $C_{14}H_{14}ClN_3O_3$

| | | | |
|---|---|---|---|
| Calculé: | C 54,64 | H 4,59 | N 13,65 |
| Trouvé: | C 54,79 | H 4,52 | N 13,69 |

*Exemple 31*

[(éthoxycarbonyl-3 chloro-6) quinolyl-4] amino-2 N,N-dibutylacétamide; dérivé N° 31.
($R_1 = R_2 = C_4H_9$; $R_3 = OC_2H_5$; $R_4 = H$, $X = Cl$)
Fusion = 116°C (rendement 60%)

Analyse: $C_{22}H_{30}ClN_3O_3$

| | | | |
|---|---|---|---|
| Calculé: | C 62,92 | H 7,20 | N 10,01 |
| Trouvé: | C 62,91 | H 7,18 | N 10,31 |

*Exemple 32*

{[(éthoxycarbonyl-3 méthoxy-6 quinolyl-4) amino] -2 acétyl}-1-pipéridine; dérivé N° 32.

(N$<^{R_1}_{R_2}$ = 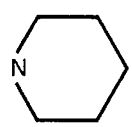, $R_3 = OC_2H_5$, $R_4 = H$; $X = OCH_3$)

Fusion = 144°C (rendement 67%)

Analyse: $C_{20}H_{25}N_3O_4$

| | | | |
|---|---|---|---|
| Calculé: | C 64,67 | H 6,78 | N 11,31 |
| Trouvé: | C 64,53 | H 6,81 | N 11,49 |

*Exemple 33*

{[(éthoxycarbonyl-3 éthoxy-6 quinolyl-4) amino] -2 acétyl}-1-pipéridine; dérivé N° 33.

(N$<^{R_1}_{R_2}$ = 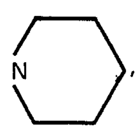, $R_3 = OC_2H_5$; $R_4 = H$; $X = OC_2H_5$)

Fusion = 150°C (rendement 30%)

Analyse: $C_{21}H_{27}N_3O_4$

| | | | |
|---|---|---|---|
| Calculé: | C 65,43 | H 7,06 | N 10,90 |
| Trouvé: | C 65,38 | H 7,26 | N 10,86 |

*Exemple 34*

[(éthoxycarbonyl-3 chloro-6) quinolyl-4] amino-2 N,N-diisopropylacétamide; dérivé N° 34.

$$(R_1 = R_2 = CH\Big\langle{}^{CH_3}_{CH_3},\ R_3 = OC_2H_5,\ R_4 = H,\ X = Cl)$$

Fusion = 170°C (rendement 40%)

Analyse: $C_{20}H_{26}ClN_3O_3$

| | | | |
|---|---|---|---|
| Calculé: | C 61,30 | H 6,69 | N 10,72 |
| Trouvé: | C 61,30 | H 6,70 | N 10,76 |

*Exemple 35*

[(éthoxycarbonyl-3 chloro-6) quinolyl-4] amino-2 N-propyl N-phényl acétamide; dérivé N° 35.
$(R_1 = C_3H_7;\ R_2 = C_6H_5;\ R_3 = OC_2H_5;\ R_4 = H;\ X = Cl)$
Fusion = 147°C (rendement 35%)

Analyse: $C_{23}\ H_{24}ClN_3O_3$

| | | | |
|---|---|---|---|
| Calculé: | C 64,86 | H 5,68 | N 9,86 |
| Trouvé: | C 64,78 | H 5,63 | N 9,75 |

*Exemple 36*

[(éthoxycarbonyl-3 chloro-6) quinolyl-4] amino-2 N-méthyl N-(propyne-2 yl) acétamide; dérivé N° 36.
$(R_1 = CH_3;\ R_2 = CH_2\text{-}C{=}CH;\ R_3 = OC_2H_5;\ R_4 = H, X = Cl)$
Fusion = 146°C (rendement 60%)

Analyse: $C_{18}H_{18}ClN_3O_3$

| | | | |
|---|---|---|---|
| Calculé: | C 60,09 | H 5,04 | N 11,68 |
| Trouvé: | C 59,89 | H 5,01 | N 11,61 |

*Exemple 37*

[(éthoxycarbonyl-3 chloro-6) quinolyl-4] amino-2 N-méthyl N-benzyl acétamide; dérivé N° 37.
$(R_1 = CH_3;\ R_2 = CH_2C_6H_5;\ R_3 = OC_2H_5;\ R_4 = H;\ X = Cl)$
Fusion: 134°C (rendement 45%)

Analyse: $C_{22}H_{22}ClN_3O_3$

| | | | |
|---|---|---|---|
| Calculé: | C 64,15 | H 5,38 | N 10,20 |
| Trouvé: | C 63,88 | H 5,39 | N 10,12 |

*Exemple 38*

{[(éthoxycarbonyl-3 chloro-6 quinolyl-4) amino] -2 acétyl}-1-méthyl-3 pipéridine; dérivé N° 38.

$(N\langle{}^{R_1}_{R_2}$ = 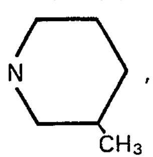 , $R_3 = OC_2H_5;\ R_4 = H,\ X = Cl,$

Fusion: 140°C (rendement 28%)

Analyse: $C_{20}\ H_{24}ClN_3O_3$

| | | | |
|---|---|---|---|
| Calculé: | C 61,61 | H 6,20 | N 10,78 |
| Trouvé: | C 61,84 | H 6,35 | N 10,48 |

*Exemple 39*

{[(éthoxycarbonyl-3 trifluorométhyl-6 quinolyl-4) amino]-2 acétyl}-1-méthyl-3- pipéridine; dérivé N° 39.

$(N\langle{}^{R_1}_{R_2}$ = 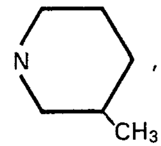 , $R_3 = OC_2H_5;\ R_4 = H;\ X = CF_3)$

Fusion: 106°C (rendement 75%)

Analyse: $C_{21}H_{24}F_3N_3O_3$

| | | | |
|---|---|---|---|
| Calculé: | C 59,57 | H 5,71 | N 9,92 |
| Trouvé: | C 59,46 | H 5,74 | N 9,61 |

*Exemple 40*

{[(éthoxycarbonyl-3 méthyl-6 quinolyl-4) amino] -2 acétyl}-1 méthyl-3 pipéridine; dérivé N° 40.

$(N\langle{}^{R_1}_{R_2}$ = 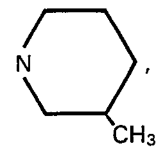 , $R_3 = OC_2H_5;\ R_4 = H,\ X = CH_3)$

Fusion: 130°C (rendement 25%)

Analyse: $C_{21}H_{27}N_3O_3$

| | | | |
|---|---|---|---|
| Calculé: | C 68,27 | H 7,37 | N 11,37 |
| Trouvé: | C 68,39 | H 7,36 | N 11,37 |

*Exemple 41*

[(éthoxycarbonyl-3 chloro-6) quinolyl-4] amino-2 N-méthyl acétamide; dérivé N° 41.
$(R_1 = CH_3;\ R_2 = H;\ R_3 = OC_2H_5;\ R_4 = H;\ X = Cl)$
Fusion: 200°C (rendement 30%)

Analyse: $C_{15}H_{16}ClN_3O_3$

| | | | |
|---|---|---|---|
| Calculé: | C 55,99 | H 5,01 | N 13,06 |
| Trouvé: | C 55,60 | H 4,85 | N 12,88 |

*Exemple 42*

[(éthoxycarbonyl-3 chloro-6) quinolyl-4] amino-2 N-propylacétamide; dérivé N° 42.
$(R_1 = C_3H_7;\ R_2 = H;\ R_3 = OC_2H_5;\ R_4 = H;\ X = Cl)$
Fusion: 179°C (rendement 48%)

Analyse: $C_{17}H_{20}ClN_3O_3$

| | | | |
|---|---|---|---|
| Calculé: | C 58,37 | H 5,76 | N 12,01 |
| Trouvé: | C 58,50 | H 5,71 | N 12,12 |

*Exemple 43*

[(carboxy-3 chloro-6) quinolyl-4] amino-2 N,N-diéthylacétamide; dérivé N° 43.
$(R_1 = R_2 = C_2H_5;\ R_3 = OH;\ R_4 = H;\ X = Cl)$

7,6 g (0,0203 mole) de [(éthoxycarbonyl-3 chloro-6) quinolyl-4] amino-2 N,N-diéthylacétamide (exemple 1) et 20,3 ml (0,0203 mole de soude (N) sont chauffés au reflux 1 heure. Après lavage au toluène, la solution est amenée à pH 7 avec HCl(N). Les cristaux obtenus sont filtrés, lavés à l'eau et recristallisés dans un mélange de dioxanne-acide acétique (8-2). On obtient l'acide attendu par séchage: cristaux incolores.
Fusion: 260°C (rendement 76%)

Analyse: $C_{16}\ H_{18}N_3ClO_3$

| | | | |
|---|---|---|---|
| Calculé: | C 57,23 | H 5,40 | N 12,51 |
| Trouvé: | C 57,25 | H 5,43 | N 12,49 |

*Exemple 44*

[(carboxy-3 méthyl-6) quinolyl-4] amino-2 N,N-diéthylacétamide; dérivé N° 44.
($R_1=R_2=C_2R_5$; $R_3=OH$; $R_4=H$; $X=CH_3$)

2,48 g (0,0065 mole) de chlorhydrate de [(éthoxycarbonyl-3 méthyl-6) quinolyl-4] amino-2 N,N--diéthylacétamide (exemple 3) dans 40 ml d'eau et 13 ml de soude 2N (0,026 mole) sont portés au reflux pendant deux heures. A température ambiante, la solution est filtrée puis acidifiée (pH 7) par HCl (N). L'acide attendu précipite: cristaux incolores F > 260°C (rendement 70%).

Analyse: $C_{17}H_{21}N_3O_3H_2O$

| | | | |
|---|---|---|---|
| Calculé: | C 61,24 | H 6,95 | N 12,60 |
| Trouvé: | C 61,49 | H 6,73 | N 12,43 |

Ont été encore préparés selon le procédé de l'exemple 43:

*Exemple 45*

{[(carboxy-3 chloro-6 quinolyl-4) amino] -2 acétyl}-1-homopipéridine; dérivé N° 45.

($N<^{R_1}_{R_2}$ = 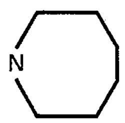, $R_3=OH$; $R_4=H$; $X=Cl$)

F > 260°C (rendement 65%)

Analyse: $C_{18}H_{20}ClN_3O_3$

| | | | |
|---|---|---|---|
| Calculé: | C 59,75 | H 5,57 | N 11,61 |
| Trouvé: | C 59,58 | H 5,57 | N 11,64 |

*Exemple 46*

[(carboxy-3 chloro-6) quinolyl-4] amino-2 N,N-dipropylacétamide; dérivé N° 46.
($R_1=R_2=C_3H_7$; $R_3=OH$; $R_4=H$; $X=Cl$)
Fusion > 260°C (rendement 42%)

Analyse: $C_{18}H_{22}ClN_3O_3$

| | | | |
|---|---|---|---|
| Calculé: | C 59,42 | H 6,10 | N 11,55 |
| Trouvé: | C 59,18 | H 6,21 | N 11,26 |

*Exemple 47*

[(carboxy-3 bromo-6) quinolyl-4] amino-2 N,N-dipropylacétamide, mono hydrate; dérivé N° 47.

($R_1=R_2=C_3H_7$; $R_3=OH$; $R_4=H$; $X=Br$)
Fusion > 260°C (rendement 80%)

Analyse: $C_{18}H_{22}BrN_3O_3$, $H_{20}$

| | | | |
|---|---|---|---|
| Calculé: | C 50,71 | H 5,67 | N 9,85 |
| Trouvé: | C 50,90 | H 5,68 | N 10,03 |

*Exemple 48*

[(isopropyloxycarbonyl-3 chloro-6) quinolyl-4] amino-2 N,N-diéthylacétamide; dérivé N° 48.
($R_1=R_2=C_2H_5$; $R_3=OCH\ (CH_3)_2$; $R_4=H$; $X=Cl$)

1) Dichloro-4,6 quinoline carboxylate d'isopropyle-3.

A une solution du chlorure de l'acide dichloro-4,6 quinoline carboxylique-3 (0,014 mole) (préparé selon C.J. OHNMACHT et al., J. Med. Chem., 1971, 14, 1, 17-24) dans le dioxane (100 ml) est ajoutée goutte à goutte à 10°C une solution d'isopropylate de sodium (0,014 mole de Na). Le mélange est agité une nuit à l'ambiante. Après évaporation des solvants le résidu est hydrolysé. On extrait au dichlorométhane, sèche sur sulfate de sodium et évapore le solvant. Le produit brut est filtré sur silice H avec le mélange cyclohexaneacétate d'éthyle (7-3). Les cristaux obtenus sont séchés sous vide.
Fusion: 66°C (rendement 36%).

2) [(isopropyloxycarbonyl-3 chloro-6) quinolyl-4] amino-2 N,N-diéthylacétamide.

Un mélange du produit précédent (1,3 g 0,0046 mole) de chlorhydrate d'amino-2 N,N-diéthylacétamide (0,84 g 0,00506 mole) et de triéthylamine (1,3 ml 0,01 mole) dans l'éthanol (30 ml) est porté 6 heures au reflux. Après évaporation du solvant, le résidu est dissous dans le dichlorométhane et lavé à l'eau. Après séchage sur sulfate de sodium et évaporation du solvant, les cristaux obtenus sont lavés à l'éther isopropylique et recristallisés dans un mélange éther isopropylique-acétate d'éthyle (6-4): cristaux incolores.
Fusion: 138°C (rendement 35%)

Analyse: $C_{19}H_{24}ClN_3O_3$

| | | | |
|---|---|---|---|
| Calculé: | C 60,39 | H 6,40 | N 11,12 |
| Trouvé: | C 60,62 | H 6,37 | N 11,30 |

*Exemple 49*

[(butoxycarbonyl-3 chloro-6) quinolyl-4] amino-2 N,N-diéthylacétamide; dérivé N° 49.
($R_1=R_2=C_2H_5$; $R_3=OC_4H_9$; $R_4=H$; $X=Cl$)
Cristaux orangés
Fusion: 120°C

Ce composé a été préparé selon le procédé décrit dans l'exemple 48 ainsi que les suivants.

Analyse: $C_{20}H_{26}ClN_3O_3$

| | | | |
|---|---|---|---|
| Calculé: | C 61,30 | H 6,69 | N 10,72 |
| Trouvé: | C 61,19 | H 6,69 | N 10,80 |

*Exemple 50*

[(benzyloxycarbonyl-3 chloro-6) quinolyl-4] amino-2 N,N-diéthylacétamide; dérivé N° 50.
($R_1=R_2=C_2H_5$; $R_3=OCH_2C_6H_5$; $R_4=H$; $X=Cl$)
Fusion: 146°C (rendement 36%)

Analyse: $C_{23}\ H_{24}ClN_3O_3$

| | | | |
|---|---|---|---|
| Calculé: | C 64,86 | H 5,68 | N 9,87 |
| Trouvé: | C 64,70 | H 5,73 | N 9,87 |

*Exemple 51*

{N'-[(benzyloxycarbonyl-3 chloro-6) quinolyl-4]-N'--méthyl amino} -2 N,N-diéthylacétamide; dérivé N° 51.
($R_1=R_2=C_2H_5$; $R_3=OCH_2C_6H_5$; $R_4=CH_3$; $X=Cl$)
Fusion: 92°C (rendement 63%)

Analyse: $C_{24}H_{26}ClN_3O_3$

| | | | |
|---|---|---|---|
| Calculé: | C 65,52 | H 5,96 | N 9,55 |
| Trouvé: | C 65,25 | H 5,73 | N 9,72 |

*Exemple 52*

{N'-[(benzyloxycarbonyl-3 chloro-6) quinolyl-4] N'-méthyl amino} -2 N,N-dipropylacétamide; dérivé N° 57.
($R_1 = R_2 = C_3H_7$; $R_3 = OCH_2C_6H_5$; $R_4 = CH_3$; X = Cl)
Fusion: 60°C (rendement 42%)

Analyse: $C_{26}H_{30}ClN_3O_3$

| | | | |
|---|---|---|---|
| Calculé: | C 66,73 | H 8,46 | N 8,98 |
| Trouvé: | C 66,73 | H 8,48 | N 8,96 |

*Exemple 53*

{N'-[(benzyloxycarbonyl-3 méthyl-6) quinolyl-4] N'-méthyl amino} -2 N,N-dipropylacétamide; dérivé N° 53.
($R_1 = R_2 = C_3H_7$; $R_3 = OCH_2C_6H_5$; $R_4 = CH_3$; X = $CH_3$)
Huile jaune (rendement 55%)

Analyse: $C_{27}H_{33}N_3O_3$

| | | | |
|---|---|---|---|
| Calculé: | C 72,46 | H 7,43 | N 9,39 |
| Trouvé: | C 72,13 | H 7,74 | N 9,49 |

*Exemple 54*

[(butyloxycarbonyl-3 chloro-6) quinolyl-4] amino-2 N,N-dipropylacétamide; dérivé N° 54.
($R_1 = R_2 = C_3H_7$; $R_3 = OC_4H_9$; $R_4$ = H; X = Cl)
Fusion: 111°C (rendement 25%)

Analyse: $C_{22}H_{30}ClN_3O_3$

| | | | |
|---|---|---|---|
| Calculé: | C 62,92 | H 7,20 | N 10,01 |
| Trouvé: | C 63,00 | H 7,26 | N 9,98 |

*Exemple 55*

[(N-Propylcarbamoyl-3 chloro-6) quinolyl-4] amino-2 N,N-diéthylacétamide; dérivé N° 55.
($R_1 = R_2 = C_2H_5$; $R_3$ = NH-$C_3H_7$; $R_4$ = H; X = Cl)

1) dichloro-4,6 quinoline N-propyl carboxamide-3.
A une solution de chlorure de l'acide dichloro-4,6 quinoline carboxylique-3 (0,038 mole) dans le dioxanne (100 ml) sont ajoutées goutte à goutte et simultanément à 10°C les solutions de propylamine (3,12 ml, 0,038 mole) et de triéthylamine (5,3 ml, 0,038 mole) dans le dioxanne (50 ml). Le mélange est agité une nuit à l'ambiante. Après évaporation du solvant, le résidu est hydrolysé et extrait au chlorure de méthylène. Après séchage sur sulfate de sodium et évaporation du solvant les cristaux obtenus sont recristallisés dans l'acétate d'éthyle.
Fusion: 152°C (rendement 61%)

2) [(N-propylcarbamoyl-3 chloro-6) quinolyl-4] amino-2 N,N-diéthylacétamide.
Un mélange du produit précédent (5,9 g, 0,0208 mole) de chlorhydrate d'amino-2 N,N-diéthyl acétamide (3,8 g, 0,023 mole) et de triéthylamine (5,9 ml, 0,046 mole) dans l'éthanol (100 ml) est porté 3 heures au reflux. Après évaporation du solvant, le résidu est solubilisé dans le dichlorométhane. Après lavage à l'eau, séchage sur sulfate de sodium et évaporation du solvant, le produit brut est cristallisé dans l'éther isopropylique et chromatographié sur colonne de silice avec l'acétate d'éthyle. Les cristaux obtenus sont lavés à l'éther isopropylique et recristallisés deux fois dans un mélange éther isopropylique acétate d'éthyle (5-5), cristaux incolores.
Fusion: 114°C (rendement 31%).

Analyse: $C_{19}H_{25}ClN_4O_2$

| | | | |
|---|---|---|---|
| Calculé: | C 60,55 | H 6,69 | N 14,87 |
| Trouvé: | C 60,44 | H 6,77 | N 14,94 |

*Exemple 56*

[(N-pnénylcarbamoyl-3 chloro-6) quinolyl-4] amino-2 N,N-diéthylacétamide; dérivé N° 56.
($R_1 = R_2 = C_2H_5$; $R_3$ = NH-$C_6H_5$; $R_4$ = H; X = Cl)
Cristaux incolores
Fusion: 160°C (rendement 45%)

Ce composé a été préparé selon le procédé décrit dans l'exemple 55.

Analyse: $C_{22}H_{23}ClN_4O_2$

| | | | |
|---|---|---|---|
| Calculé: | C 64,31 | H 5,64 | N 13,63 |
| Trouvé: | C 64,31 | H 5,42 | N 13,46 |

*Exemple 57*

{[N-(éthoxycarbonyl-3 chloro-6 quinolyl-4) N-méthyl amino] -2 acétyl}-4-morpholine; dérivé N° 57.

($N<^{R_1}_{R_2}$ = 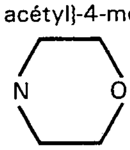 ; $R_3 = OC_2H_5$; $R_4 = CH_3$; X = Cl)

1) N-(N-benzyloxycarbonyl-sarcosyl) morpholine.
Un mélange de 12 g (0,0219 mole) de p-nitrophényl ester de N-benzyloxycarbonyl sarcosine (préparé selon A.B. MAUGER et R. WADE, J; Chem. Soc., 1965, p. 3131) de 3,8 ml de morpholine dans 75 ml d'acétate d'éthyle est agité à température ambiante pendant 24 h. Le milieu réactionnel est évaporé à sec. Le résidu d'évaporation est repris au dichlorométhane. Les phases organiques sont lavées à HCl (N), $NH_4OH$ (2N), plusieurs fois à l'eau, puis séchées sur $Na_2SO_4$ anhydre et évaporées à sec. Le résidu d'évaporation est repris à l'éther isopropylique pour donner des cristaux jaunes.
Fusion 82°C (rendement quantitatif).

2) N-sarcosyl morpholine.
Un mélange de 6,8 g (0,0219 mole) du produit précédent de 1,4 g de Pd/C à 10% dans 100 ml dde méthanol est agité à température ambiante, sous hydrogène pendant 4 h. Le milieu réactionnel est filtré sur talc. Le filtrat évaporé à sec fournit l'amide attendue.
(rendement 92%)

3) {[N-(éthoxycarbonyl-3 chloro-6 quinolyl-4) N-méthyl amino] -2 acétyl}-4-morpholine.
Un mélange de 3,87 g (0,0143 mole) de dichloro-4,6 quinoline carboxylate d'éthyle-3 de 3,4 g (1,5 éq.) du produit précédent et de 3,02 ml de triéthylamine dans 50 ml d'éthanol est chauffé au reflux pendant 5 h. Le milieu réactionnel est évaporé à sec. Le résidu d'évaporation est repris au dichlorométhane, lavé à l'eau, séché sur $Na_2SO_4$ anhydre puis évaporé à sec pour donner des cristaux jaunes qui sont filtrés sur colonne de silice (acétate d'éthyle): cristaux jaunes.
Fusion: 102°C (rendement 62%).

Analyse: $C_{19}H_{22}ClN_3O_4$

| | | | |
|---|---|---|---|
| Calculé: | C 58,24 | H 5,66 | N 10,72 |
| Trouvé: | C 58,15 | H 5,72 | N 10,72 |

*Exemple 58*

{N'-[(éthoxycarbonyl-3 chloro-6) quinolyl-4] N'-méthyl amino}-2 N,N-diéthylacétamide; dérivé N° 58.
($R_1 = R_2 = C_2H_5$; $R_3 = OC_2H_5$; $R_4 = CH_3$; X = Cl)
Cristaux jaunes.
Fusion: 118°C (rendement 55%).

Ce composé a été préparé selon le procédé décrit dans l'exemple 57.

Analyse: $C_{19}H_{24}ClN_3O_3$

| | | | |
|---|---|---|---|
| Calculé: | C 60,39 | H 6,40 | N 11,12 |
| Trouvé: | C 60,27 | H 6,42 | N 11,09 |

*Exemple 59*

{N'-[(éthoxycarbonyl-3 chloro-6) quinolyl-4] N'-méthyl amino}-2 N,N-dipropylacétamide; dérivé N° 59.
($R_1 = R_2 = C_3H_7$; $R_3 = OC_2H_5$; $R_4 = CH_3$; X = Cl)

1) N, (tert-butyloxycarbonyl) sarcosine

15 g(0,168 mole) de sarcosine, 40,3 g (0,185 mole) de $(Boc)_2O$, 168 ml (0,168 mole) de NaOH (N) 400 ml de dioxanne et 200 ml d'eau sont agités à température ambiante pendant 5 h. Le dioxanne éliminé, le résidu est repris par l'eau. La phase aqueuse est lavée au dichlorométhane puis acidifiée par HCl (6N). On extrait au dichlorométhane, lave à l'eau et sèche sur sulfate de sodium. Le solvant éliminé, on récueille une huile jaune claire (rendement 89%) qui est utilisée pour l'étape suivante.

2) p-nitrophényl ester du N-(tert-butyloxycarbonyl) sarcosine.

Au mélange de 3,6 g (0,0189 mole) de l'huile précédente, 2,8 g (1,07 mole) de p-nitrophénol 45 ml d'acétate d'éthyle est ajouté à température ambiante 4,13 g (1,06 éq.) de N, N'-dicyclohexylcarbodiimide.
Après 24 h d'agitation, le précipité est filtré. La phase organique après extraction par NaOH (2N) est lavée à l'eau puis séchée. Après élimination du solvant, on recueille une huile claire,
(rendement 88%)

3) N'-tert-butyloxycarbonyl N'-méthyl amino-2 N,N-dipropylacétamide.

Un mélange de p-nitrophényl ester de N-(tert-butyloxycarbonyl) sarcosine (10 g, 0,032 mole) et de dipropylamine (8,8 ml, 0,064 mole) dans l'acétate d'éthyle (60 ml) est agité 24 h à 25°C. Après addition de dichlorométhane, lavage avec HCl (2N), $NH_4OH$ (2N) et l'eau, séchage sur sulfate de sodium et évaporation du solvant le produit brut est séché sous vide (rendement quantitatif).

4) N'-méthyl amino-2 N,N-dipropylacétamide.

Au mélange du produit précédent (7,7 g, 0,034 mole) dans le chloroforme (30 ml) est ajouté, goutte à goutte, à 5°C l'acide trifluoroacétique (20 ml, 0,118 mole). La solution est agitée 2 h à l'ambiante. Après évaporation du solvant, le produit brut est hydrolysé et extrait à l'éther éthylique. La phase aqueuse, rendue alcaline est extraite au dichlorométhane, séchée sur sulfate de sodium. Après évaporation du solvant, le produit brut est séché sous vide (rendement 48%).

5) {N'[(éthoxycarbonyl-3 chloro-6) quinolyl-4] N'-méthyl amino}-2 N,N-dipropylacétamide.

Un mélange de dichloro-4,6 quinoline carboxylate-3 d'éthyle (1,7 g, 0,0097 mole), du produit précédent (2,5 g, 0,0145 mole) et de triéthylamine (2,05 ml, 0,0145 mole) dans l'éthanol (30 ml) est porté 4 h au reflux. Après évaporation du solvant, le produit est repris par le dichlorométhane. La phase organique est lavée à l'eau, séchée puis évaporée sous vide. On cristallise le produit attendu dans l'éther isopropylique.
Cristaux incolores.
Fusion: 76°C (rendement 33%).

Analyse: $C_{21}H_{28}ClN_3O_3$

| | | | |
|---|---|---|---|
| Calculé: | C 62,14 | H 6,95 | N 10,35 |
| Trouvé: | C 62,29 | H 7,25 | N 10,40 |

*Exemple 60*

{N'-[(éthoxycarbonyl-3 méthyl-6) quinolyl-4] N'-méthyl-amino}-2 N,N-diéthylacétamide; dérivé N° 60.
($R_1 = R_2 = C_2H_5$; $R_3 = OC_2H_5$; $R_4 = CH_3$; H = $CH_3$
Cristaux incolores.
Fusion: 116,5°C (rendement 40%)

Ce composé a été préparé selon le procédé décrit à l'exemple 59 ainsi que les suivants.

Analyse: $C_{20}H_{27}N_3O_3$

| | | | |
|---|---|---|---|
| Calculé: | C 67,20 | H 7,61 | N 11,76 |
| Trouvé: | C 67,08 | H 7,48 | N 11,89 |

*Exemple 61*

{N'-[(éthoxycarbonyl-3 méthyl-6) quinolyl-4] N'-méthyl amino}-2 N,N-dipropylacétamide, chlorhydrate; dérivé N° 61.
($R_1 = R_2 = C_3H_7$; $R_3 = OC_2H_5$; $R_4 = CH_3$; X = $CH_3$)
Fusion: 169°C (rendement 70%)

Analyse: $C_{22}$ $H_{31}N_3O_3$, HCl

| | | | |
|---|---|---|---|
| Calculé: | C 62,62 | H 7,64 | N 9,96 |
| Trouvé: | C 62,33 | H 7,67 | N 9,87 |

*Exemple 62*

{N'-[(éthoxycarbonyl-3 trifluorométhyl-6] quinolyl-4] N'-méthyl amino} -2 N,N-dipropylacétamide; dérivé N° 62.
($R_1 = R_2 = C_3H_7$; $R_3 = OC_2H_5$; $R_4 = CH_3$; X = $CF_3$)
Fusion: 80°C (rendement 50%)

Analyse: $C_{22}H_{28}F_3N_3O_3$

| | | | |
|---|---|---|---|
| Calculé: | C 60,13 | H 6,42 | N 9,56 |
| Trouvé: | C 60,13 | H 6,70 | N 9,24 |

*Exemple 63*

{N'-[(éthoxycarbonyl-3 bromo-6) quinolyl-4] N'-méthyl amino} -2 N,N-dipropylacétamide; dérivé N° 63. ($R_1 = R_2 = C_3H_7$; $R_3 = OC_2H_5$; $R_4 = CH_3$; X = Br)
Fusion: 82°C (rendement 28%)

Analyse: $C_{21}H_{28}BrN_3O_3$

| | | | |
|---|---|---|---|
| Calculé: | C 55,99 | H 6,26 | N 9,32 |
| Trouvé: | C 56,21 | H 6,45 | N 9,46 |

*Exemple 64*

{N'-[(éthoxycarbonyl-3 chloro-6) quinolyl-4] N'-méthyl amino} -2 N-propylacétamide; dérivé N° 64. ($R_1 = C_3H_7$; $R_2 = H$; $R_3 = OC_2H_5$; $R_4 = CH_3$; X = Cl)
Fusion: 154°C (rendement 75%)

Analyse: $C_{18}H_{22}ClN_3O_3$

| | | | |
|---|---|---|---|
| Calculé: | C 59,42 | H 6,09 | N 11,55 |
| Trouvé: | C 59,55 | H 6,03 | N 11,58 |

*Exemple 65*

{N'-[(éthoxycarbonyl-3 chloro-6) quinolyl-4] N'-propyl amino} -2 N,N-diéthylacétamide; dérivé N° 65. ($R_1 = R_2 = C_2H_5$; $R_3 = OC_2H_5$; $R_4 = C_3H_7$; X = Cl)

1) Chloro-2 N,N-diéthylacétamide.

A une solution de chlorure de chloroacétyle (10 g, 0,0885 mole) dans le dioxanne (ml) sont ajoutées goutte à goutte et simultanément à 10°C les solutions de diéthylamine (9,15 ml, 0,0885 mole) et de triéthylamine (12,3 ml, 0,0885 mole) dans le dioxanne (20 ml). Après agitation une nuit à l'ambiante et évaporation du solvant le résidu est hydrolysé et extrait au dichlorométhane. Après lavages successifs à l'acide chlorhydrique et au bicarbonate de sodium à 5%, séchage sur sulfate de sodium et évaporation du solvant. Le produit brut est filtré sur silice H avec l'acétate d'éthyle (rendement 61%).

2) N'-propylamino-2 N,N-diéthylacétamide.

Un mélange du produit précédent (6 g, 0,039 mole) de propylamide (3,6 ml, 0,043 mole) et de triéthylamine (6 ml, 0,043 mole) dans l'éthanol (60 ml) est porté 3 h au reflux. Après évaporation du solvant, le produit brut est solubilisé dans le dichlorométhane. Après lavage à l'eau, séchage sur sulfate de sodium et évaporation du solvant, l'huile obtenue est séchée sous vide (rendement 56%).

3) {N'-[(éthoxycarbonyl-3 chloro-6) quinolyl-4] N'-propylamino} -2 N,N-diéthylacétamide.

Un mélange du produit précédent (3,7 g, 0,021 mole) de dichloro-4,6 quinoline carboxylate d'éthyle-3 (4,9 mg, 0,019 mole) et de triéthylamine (3 ml, 0,021 mole) dans l'éthanol (40 ml) est porté 3 h au reflux. Après évaporation du solvant, lavage à l'eau, le produit brut est purifié par chromatographie sur silice (cyclohexane-acétate d'éthyle (5-5). Le produit attendu est recristallisé dans éther de pétrole.
Cristaux jaunes.
Fusion: 85°C (rendement 30%).

Analyse: $C_{21}H_{28}ClN_3O_3$

| | | | |
|---|---|---|---|
| Calculé: | C 62,14 | H 6,95 | N 10,35 |
| Trouvé: | C 62,19 | H 7,12 | N 10,25 |

*Exemple 66*

[(acétyl-3-méthyl-6) quinolyl-4] amino-2 N,N-dipropylacétamide; dérivé N° 66.
$R_1 = R_2 = C_3H_7$; $R_3 = CH_3$; $R_4 = H$; $X = CH_3$

1) *Acetyl-3-chloro-4 méthyl-6 quinoléine*

L'acétyl-3 hydroxy-4 méthyl-6 quinoléine (0,02 mole), préparé selon R.K. MAPARA et C.M. DESAI (CA 50 1011 b) est ajouté à 40 ml d'oxychlorure de phosphore fraichement distillé. Le mélange est porté 10 minutes au reflux. La solution ramenée à température ambiante est jetée sur un mélange glace-soude. Après extraction au dichlorométhane, la phase organique est décantée, lavée jusqu'à neutralité, séchée et concentrée. L'acétyl-3 chloro-4 méthyl-6 quinoléine (F = 145°C) est utilisé sans autre purification pour l'étape suivante.

2) *[(Acétyl-3 méthyl-6) quinolyl-4] amino-2 N,N--dipropylacétamide*

Au composé précédent (0,006 mole) dissous dans 60 ml d'éthanol absolu est ajouté 0,0066 mole de chlorhydrate d'amino-2 N,N-dipropylacétamide et 0,013 mole de triéthylamine. Cette solution est portée douze heures au reflux. Après élimination de l'éthanol, le résidu est lavé à l'eau puis purifié par chromatographie sur colonne de silice (éluant toluène éthanol 95-5) et recristallisation dans le toluène: cristaux incolores.
F: 163°C (rendement 75%)

Analyse: $C_{20}H_{27}N_3O_2$

| | | | |
|---|---|---|---|
| Calculé: | C 70,35 | H 7,97 | N 12,31 |
| Trouvé: | C 70,34 | H 7,98 | N 12,32 |

Les résultats des essais toxicologiques et pharmacologiques qui sont rapportés ci-après ont permis de mettre en évidence la bonne tolérance des dérivés de l'invention ainsi que leurs intéressantes propriétés.

L'invention a donc encore pour objet un médicament ayant en particulier des activités psychotropes, caractérisé en ce qu'il contient, à titre de principe actif un dérivé de formule (I) ou un sel d'addition avec une base ou un acide minéral ou organique pharmaceutiquement acceptable.

*Etude toxicologique*

Cette étude a porté sur les toxicités aigüe, chronique, sub-chronique et retardée. Effectués sur diverses espèces animales, les essais ont mis en lumière la faible toxicité et la bonne tolérance des dérivés de l'invention.

L'évaluation de la toxicité aigüe chez la souris a permis de montrer que l'administration orale de doses de 1500 mg/kg de poids pondéral était parfaitement tolérée: à titre indicatif, la DL 50 est de 1800 mg/kg pour le dérivé N° 1, de 1650 mg pour le dérivé N° 3 et de 2200 pour le dérivé N° 43.

*Etude pharmacologique*

Les résultats des essais pharmacologiques qui

sont rapportés ci-après ont mis en évidence les intéressantes propriétés psychotropes des dérivés de l'invention.

Ces dérivés qui possèdent une remarquable affinité pour les récepteurs des benzodiazépines présentent des propriétés anticonvulsivantes, sédatives, hypnogènes et anxiolytiques.

1) *Affinité pour les récepteurs des benzodiazépines*

L'étude a été effectuée selon la technique de WASTEK et al., (Europ. J. Pharmacol., 1978, 50, 445-447) légèrement modifiée.

Sur le rat sacrifié par décapitation, le cerveau est prélevé, broyé, mis en suspension, centrifugé et remis en suspension.

Des échantillons de cette suspension sont mis à incuber en présence de 3H flunitrazépam seul ou en présence de concentrations croissantes du composé à tester.

On détermine alors, par scintillation liquide, la radioactivité retenue sur les membranes.

On détermine ainsi la concentration en produit inhibant 50% de la liaison spécifique du 3H flunitrazépam (C.I. 50).

A titre de comparaison, le chlordiazépoxide possède une C.I. 50 (exprimée en nM) de 950 et le diazépam de 10.

Les C.I. 50 ainsi déterminées pour les dérivés de l'invention sont rapportés ci-après:

| Dérivé | C.I. 50 nM | Dérivé | C.I. 50 nM |
|---|---|---|---|
| 1 | 85 | 26 | 333 |
| 2 | 41 | 27 | 38 |
| 3 | 55 | 28 | 600 |
| 4 | 97 | 29 | 687 |
| 5 | 97 | 30 | 700 |
| 6 | 155 | 33 | 54 |
| 7 | 639 | 34 | 48 |
| 8 | 133 | 35 | 27 |
| 9 | 900 | 36 | 99 |
| 10 | 1560 | 38 | 65 |
| 12 | 16 | 41 | 440 |
| 13 | 25 | 43 | 7 |
| 14 | 17 | 44 | 46 |
| 15 | 483 | 45 | 7 |
| 16 | 38 | 46 | 1 |
| 17 | 8 | 47 | 1 |
| 18 | 26 | 48 | 69 |
| 19 | 28 | 49 | 61 |
| 20 | 40 | 50 | 27 |
| 21 | 28 | 52 | 727 |
| 22 | 14 | 53 | 638 |
| 23 | 100 | 55 | 150 |
| 24 | 251 | 56 | 136 |
| 25 | 148 | 66 | 92 |

2) *Activité anticonvulsivante*

On a étudié l'effet de protection produit par les composés de l'invention contre les crises au pentétrazole.

Ce test a été réalisé selon la méthode de EVERETT et RICHARDS (J. Pharm. Exp. Ther., 1944, 81, 402-407). Les produits à tester ainsi que les produits de référence ont été administrés par voie orale (en suspension dans la gomme arabique) à des lots de 10 souris, 30 minutes avant le pentétrazole (125 mg/kg par voie sous-cutanée). On note dans chaque lot le nombre d'animaux qui ne présentent pas de crises toniques pendant les 30 minutes qui suivent l'administration de l'agent convulsivant. Les doses efficaces 50 (DESO) et leur limite de confiance à 95% ont été calculées par la méthode de FINNEY (1971).

Les résultats sont rassemblés dans le tableau cidessous:

| Dérivé | DESO (Intervalle de confiance) mg/kg |
|---|---|
| 1 | 49 (30-88) |
| 1 maléate | 27 (18-36) |
| 5 | 50 (30-60) |
| 10 | 68 (50-90) |
| 12 | 10 (05-16) |
| 13 | 25 (13-35) |
| 14 | 40 (31-57) |
| 17 | 18 (15-22) |
| 25 | 33 (24-45) |
| 26 | 50 (27-96) |
| 29 | 38 (16-59) |
| 38 | 46 (29-70) |
| 41 | 46 (23-79) |
| 49 | 54 (36-87) |
| 52 | 31 (19-79) |
| 58 | 44 (35-56) |
| 59 | 16 (11-21) |
| 61 | 24 (14-38) |
| 63 | 24 (19-30) |
| Chlordiazépoxide | 7 |
| Phénobarbital | 10 |
| Méprobamate | 36 |

3) *Activité anxiolytique*

Cette activité a été étudiée selon le test de la néophobie alimentaire. En effet, une inhibition prononcée de la prise alimentaire peut-être obtenue chez la souris lorsque nourriture et enceinte sont toutes deux inconnues de l'animal (R.J. STEPHENS, Brit. J. Pharmacol., 1973, 47, 145 P). Tous les anxiolytiques s'opposent spécifiquement aux phénomènes de néophobie.

Les souris, à jeun depuis 16 à 20 heures sont isolées, 30 minutes après traitement par les voies orales ou sous-cutanées, par les dérivés à tester et les produits de référence, dans une enceinte translucide et mises en présence d'une nourriture nouvelle pendant 5 minutes.

Les quantités consommées sont pondérées par le poids de l'animal.

On calcule ainsi dans chaque lot, la consommation moyenne ainsi que le pourcentage de variation par rapport au lot témoin.

Les résultats sont rassemblés dans le tableau suivant:

| Dérivé | Dose mg/kg | Voie | Pourcentage d'augmentation |
|---|---|---|---|
| 12 | 32 | VO | 75 |
| 13 | 16 | VO | 75 |
| 17 | 32 | SC | 75 |
| 43 | 64 | SC | 75 |
| 44 | 32 | SC | 50 |
| 46 | 16 | SC | 75 |
| 58 | 64 | SC | 80 |
| 59 | 32 | VO | 146 |
| Chlor-diazépoxide | 16 | VO | 73 |
| Diazepam | 4 | VO | 80 |
| Méprobamate | 60 | VO | 95 |

4) *Activité hypnogène*

L'activité hypnogène des composés de la présente invention a été évaluée selon la méthode de JANSEN (J. Med. Pharm. Chem., 1959, 1, 281).

Après traitement par voie i.p., chaque souris est placée délicatement sur le dos. L'animal est considéré en état de sommeil s'il ne se retourne pas et reste sur son dos pendant au moins 30 sec. On note dans chaque lot (10 souris par dose) le pourcentage d'animaux en sommeil. La dose hypnotique 50 et son intervalle de confiance à 95% a été calculée selon la méthode de FINNEY (1971).

Les résultats sont rassemblés dans le tableau ci-après:

| Dérivé | Dose hypnotique 50 (mg/kg) (intervalle de confiance) | |
|---|---|---|
| | i.p. | i.v. |
| 3 | 27 (22-33) | 7 (6-9) |
| 8 | 18 (13-24) | 5 (3-7) |
| 14 | 25 (21-29) | 5 (4-7) |
| 4 | 27 (21-31) | 5 (4-6) |
| 5 | 25 (21-28) | 9 (7-10) |
| 12 (Phosphate) | 38 (31-47) | 2 (1-4) |
| 12 (Sulfate) | 30 (14-39) | 6 (4-15) |
| 33 | 15 (11-19) | 5 (4-6) |
| 38 | 28 (22-37) | 9 (7-10) |

A titre de comparaison, la dose hypnotique 50 du Thiopental sodique, agent hypnogène très actif est de 17 mg/kg et celle di Midazolam est de 23 mg/kg (L. PIERI et al. Arzneim. Forsh., 1981, 31(11) N° 12a, 2180-2201).

Les études toxicologiques et pharmacologiques qui viennent d'être rapportées ont mis en évidence la faible toxicité des composés de l'invention et leur bonne tolérance, ainsi que leurs intéressantes propriétés psychotrope qui les rendent très utiles en thérapeutique humaine et vétérinaire.

Le médicament de l'invention peut être présenté pour l'administration orale sous forme de comprimés, comprimés dragéifiés, capsules, gouttes, granulés ou sirop.

Il peut être aussi présenté pour l'administration rectale sous forme de suppositoires et pour l'administration parentérale sous forme de soluté injectable.

Chaque dose unitaire contient avantageusement de 5 mg à 300 mg de principe actif, les doses administrables journellement pouvant varier de 5 mg à 500 mg de principe actif en fonction de l'âge du patient et de la sévérité de l'affection traitée.

On donnera ci-après, à titre d'exemples non limitatifs, quelques formulations pharmaceutiques du médicament de l'invention:

1. Comprimés - dérivé N° 25: 30 mg
   Excipient: silicate de calcium, polyvinyl pyrrolidone, stéarate de magnésium, talc, amidon de maïs, oxyde de titane, acétylphtalate de cellulose.
2. Comprimés dragéifiés - derivé N° 12: 15 mg
   Excipient: levilite, amidon, gélatine, stéarate de magnésium, gomme laque, talc, gomme arabique, saccharose, oxyde de titane.
3. Gélules - dérivé N° 59: 20 mg
   Excipient: talc, stéarate de magnesium.
4. Sirop - dérivé N° 14: 300 mg
   Excipient aromatisé qsp 100 ml.
5. Soluté injectable - dérivé N° 8: 25 mg
   Solvant isotonique qsp 5 ml
6. Suppositoires - dérivé N° 44: 30 mg
   Glycérides semi-synthétiques qsp 1 suppositoire.

Le médicament de l'invention est utilisé avec profit en médecine pour son activité psychotrope.

Par ses propriétés anticonvulsivante, sédative, hypnogène et anxiolytique il est indiqué, aussi bien chez l'adulte que chez l'enfant dans le traitement des états convulsifs, des états anxieux, des troubles du comportement, du stress et de l'angoisse, et aussi en tant que myorelaxant, hypno-inducteur et anesthésique.

**Revendications pour les Etats contractants:**
BE, CH, DE, FR, GB, IT, LI, LU, NL, SE

1. Dérivés quinolylglycinamides répondant à la formule générale suivante:

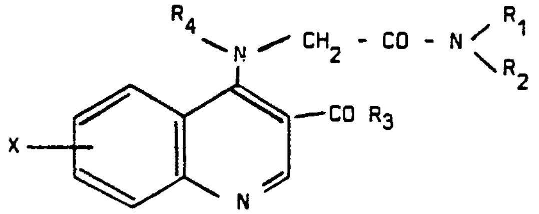

(I)

dans laquelle:

$R_1$ et $R_2$ représentent chacun indépendamment l'un de l'autre un hydrogène ou un radical alcoyle en $C_1$-$C_4$, ou bien lorsque $R_1$ est un radical alcoyle, $R_2$ peut représenter un radical cycloalcoyle, un radical phényle ou benzyle éventuellement substitués par un halogène ou un alcoyle en $C_1$-$C_4$, ou bien encore $R_1$ et $R_2$ peuvent former avec l'atome d'azote auquel ils sont rattachés un hétérocycle pouvant comporter un second hétéroatome tel que l'oxygène, le soufre ou l'azote; $R_3$ représente un hydrogène, un hydroxyle OH, un groupe alcoyle ou alcoxy en $C_1$-$C_4$, phénoxy ou benzyloxy éventuellement substitués par un halogène ou un alcoyle en $C_1$-$C_4$, ou bien $R_3$ représente un groupement -NH-$R_5$ dans lequel $R_5$ est un hydrogène, un radical alcoyle en $C_1$-$C_4$ ou phényle; $R_4$ représente un hydrogène ou un radical alcoyle en $C_1$-$C_4$; X représente un atome d'hydrogène ou un halogène, un radical alcoyle en $C_1$-$C_4$, cycloalcoyle, alcoxy, nitro, trifluorométhyle, méthylthio ainsi que leurs sels d'addition avec les acides minéraux ou organiques pharmaceutiquement acceptables et éventuellement, lorsque $R_3$ représente OH, les sels avec les bases alcalines ou organiques pharmaceutiquement acceptables.

2. Dérivés de formule (I) selon la revendication 1, caractérisés en ce que $R_1$ et $R_2$ représentent des groupes alcoyles en $C_1$-$C_4$, $R_3$ représente un groupe alcoxy en $C_1$-$C_4$ et $R_4$ représente un hydrogène.

3. Dérivés de formule (I) selon la revendication 1, caractérisé en ce que $R_1$, $R_2$ et $R_4$ représentent des groupes alcoyles en $C_1$-$C_4$, $R_3$ représente un groupe $NHR_5$ dans lequel $R_5$ est l'hydrogène ou un groupe alcoyle en $C_1$-$C_4$.

4. Dérivés de formule (I) selon la revendication 1, caractérisés en ce que N $R_1$ $R_2$ forment un hétérocyle pyrrolidino, pipéridino, homopipéridino ou morpholino pouvant porter un substituant alcoyle en $C_1$-$C_4$.

5. Dérivés de formule (I) selon la revendication 4, caractérisés en ce que le groupe N $R_1$ $R_2$ représente un groupe morpholino, $R_3$ représente l'hydrogène ou un groupe alcoxy en $C_1$-$C_4$, et $R_4$ représente l'hydrogène ou un groupe alcoyle en $C_1$-$C_4$.

6. Dérivés de formule (I) selon l'une des revendications 1 à 5, caractérisés en ce que X situé en position 6 du noyau quinoléine représente un halogène, un radical alcoyle en $C_1$-$C_4$ ou cycloalcoyle, alcoxy en $C_1$-$C_4$, nitro ou trifluorométhyle.

7. [(éthoxycarbonyl-3 cloro-6) quinolyl-4] amino-2 N,N-dipropylacétamide.

8. {N'-[(éthoxycarbonyl-3 chloro-6) quinolyl-4] N'méthyl amino} -2 N,N-dipropylacétamide.

9. Procédé de préparation des dérivés de formule (I) selon la revendication 1, caractérisé en ce que l'on fait réagir un dérivé de la glycine de formule

$$R_4\text{-HN-CH}_2\text{-CO-N}\langle^{R_1}_{R_2} \qquad \text{(II)}$$

avec une chloro-4 quinoléine de formule

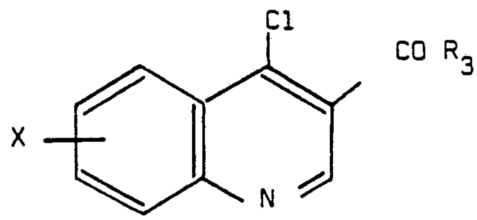

(III)

dans lesquelles $R_1$, $R_2$, $R_3$, $R_4$ et X ont les significations indiquées pour la formule (I) dans l'une des revendications 1 à 6.

10. Procédé selon la revendication 9, caractérisé en ce que la réaction de condensation est effectuée par chauffage des 2 réactifs à une température comprise entre 60°C et 130°C.

11. Procédé selon la revendication 10, caractérisé en ce que la réaction de condensation s'effectue en présence d'une base organique ou minérale.

12. Procédé selon la revendication 10, caractérisé en ce que la réaction de condensation est avantageusement réalisée dans un solvant organique tel que le toluène, l'alcool éthylique ou isopropylique.

13. Médicament caractérisé en ce qu'il contient, à titre de principe actif, un dérivé selon l'une des revendications 1 à 8.

14. Médicament selon la revendication 13, caractérisé en ce qu'il est présenté sous une forme appropriée à l'administration orale, parentérale ou rectale.

15. Médicament selon la revendication 13 ou 14, caractérisé en ce qu'il est présenté sous forme de doses unitaires contenant de 0,005 g à 0,300 g.

**Revendications pour l'Etat contractant:** AT

1. Procédé de préparation de dérivés de quinolylglycinamides répondant à la formule générale suivante:

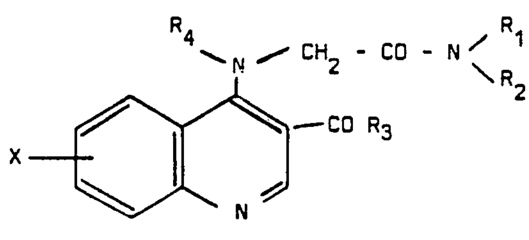

(I)

dans laquelle:

$R_1$ et $R_2$ représentent chacun indépendamment l'un de l'autre un hydrogène ou un radical alcoyle en $C_1$-$C_4$, ou bien lorsque $R_1$ est un radical alcoyle, $R_2$ peut représenter un radical cycloalcoyle, un radical phényle ou benzyle éventuellement substitués par un

halogène ou un alcoyle en $C_1$-$C_4$, ou bien encore $R_1$ et $R_2$ peuvent former avec l'atome d'azote auquel ils sont rattachés un hétérocycle pouvant comporter un second hétéroatome tel que l'oxygène, le soufre ou l'azote; $R_3$ représente un hydrogène, un hydroxyle OH, un groupe alcoyle ou alcoxy en $C_1$-$C_4$, phénoxy ou benzyloxy éventuellement substitués par un halogène ou un alcoyle en $C_1$-$C_4$, ou bien $R_3$ représente un groupement -NH-$R_5$ dans lequel $R_5$ est un hydrogène, un radical alcoyle en $C_1$-$C_4$ ou phényle; $R_4$ représente un hydrogène ou un radical alcoyle en $C_1$-$C_4$; X représente un atome d'hydrogène ou un halogène, un radical alcoyle en $C_1$-$C_4$, cycloalcoyle, alcoxy, nitro, trifluorométhyle, méthylthio ainsi que leurs sels d'addition avec les acides minéraux ou organiques pharmaceutiquement acceptables et éventuellement, lorsque $R_3$ représente OH, les sels avec les bases alcalines ou organiques pharmaceutiquement acceptables caractérisé en ce que l'on fait réagir un dérivé de la glycine de formule

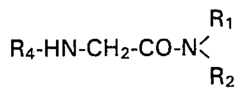

(II)

avec une chloro-4 quinoléine de formule

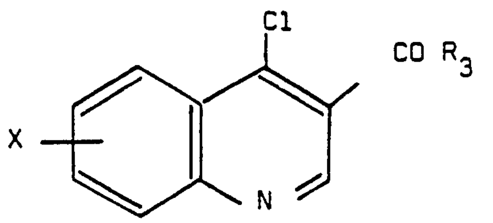

(III)

dans lesquelles $R_1$, $R_2$, $R_3$, $R_4$ et X ont les significations indiquées pour la formule (I).

2. Procédé selon la revendication 1, caractérisé en ce que $R_1$ et $R_2$ représentent des groupes alcoyles en $C_1$-$C_4$, $R_3$ représente un groupe alcoxy en $C_1$-$C_4$ et $R_4$ raprésente un hydrogène.

3. Procédé selon la revendication 1, caractérisé en ce que $R_1$, $R_2$ et $R_4$ représentent des groupes alcoyles en $C_1$-$C_4$, $R_3$ représente un groupe $NHR_5$ dans lequel $R_5$ est l'hydrogène ou un groupe alcoyle en $C_1$-$C_4$.

4. Procédé selon la revendication 1, caractérisé en ce que N $R_1$ $R_2$ forment un hétérocycle pyrrolidino, pipéridino, homopipéridino ou morpholino pouvant porter un substituant alcoyle en $C_1$-$C_4$.

5. Procédé selon la revendication 4, caractérisé en ce que le groupe N $R_1$ $R_2$ représente un groupe morpholino, $R_3$ représente l'hydrogène ou un groupe alcoxy en $C_1$-$C_4$, et $R_4$ représente l'hydrogène ou un groupe alcoyle en $C_1$-$C_4$.

6. Procédé selon l'une des revendications 1 à 5, caractérisé en ce que X situé en position 6 du noyau quinoléine représente un halogène, un radical alcoyle en $C_1$-$C_4$ ou cycloalcoyle, alcoxy en $C_1$-$C_4$, nitro ou trifluorométhyle.

7. Procédé selon l'une des revendications 1 à 6, caractérisé en ce que la réaction de condensation est effectuée par chauffage des 2 réactifs à une température comprise entre 60°C et 130°C.

8. Procédé selon l'une des revendications 1 à 9, caractérisé en ce que la réaction de condensation s'effectue en présence d'une base organique ou minérale.

9. Procédé selon l'une des revendications 1 à 8, caractérisé en ce que la réaction de condensation est avantageusement réalisée dans un solvant organique tel que le toluène, l'alcool éthylique ou isopropylique.

10. Procédé de préparation d'un médicament, caractérisé en ce qu'il contient, à titre de principe actif, un dérivé obtenu selon l'une des revendications 1 à 9.

**Patentansprüche für die Vertragsstaaten:**
BE, CH, DE, FR, GB, IT, LI, LU, NL, SE

1. Chinolylglycinamid-Derivate der folgenden allgemeinen Formel (I):

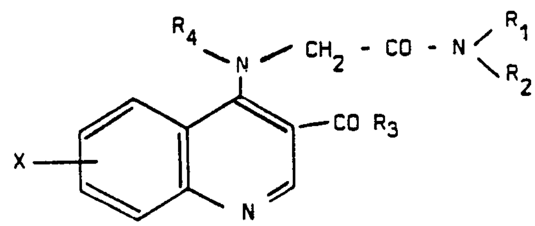

(I)

in der bedeuten:

$R_1$ und $R_2$, unabhängig voneinander, je Wasserstoff oder $C_{1-4}$-Alkyl, oder, wenn $R_1$ Alkyl ist, $R_2$ Cycloalkyl, ggf. mit einem Halogen oder einem $C_{1-4}$-Alkyl substituiertes Phenyl oder Benzyl, oder $R_1$ und $R_2$ zusammen mit dem Stickstoffatom, an das sie gebunden sind, einen Heterocyclus mit einem zweiten Heteroatom, wie Sauerstoff, Schwefel oder Stickstoff,

$R_3$ Wasserstoff, Hydroxyl, $C_{1-4}$-Alkyl oder $C_{1-4}$-Alkoxy, ggf. mit einem Halogen oder einem $C_{1-4}$-Alkyl substituiertes Phenoxy oder Benzyloxy oder -NH-$R_5$ mit $R_5$ Wasserstoff, $C_{1-4}$-Alkyl oder Phenyl,

$R_4$ Wasserstoff oder $C_{1-4}$-Alkyl,

X Wasserstoff oder Halogen, $C_{1-4}$-Alkyl, Cycloalkyl, Alkoxy, Nitro, Trifluormethyl oder Methylthio,

sowie Ihre Säureadditionssalze mit pharmazeutisch verträglichen anorganischen oder organischen Säuren und ggf., wenn $R_3$ OH ist, Salze mit pharmazeutisch verträglichen alkalischen oder organischen Basen.

2. Derivate der Formel (I) nach Anspruch 1, dadurch gekennzeichnet, daß $R_1$ und $R_2$ $C_{1-4}$-Alkyl, $R_3$ $C_{1-4}$-Alkoxy und $R_4$ Wasserstoff bedeuten.

3. Derivate der Formel (I) nach Anspruch 1, dadurch gekennzeichnet, daß $R_1$, $R_2$ und $R_4$ $C_{1-4}$-Alkyl, $R_3$ $NHR_5$ mit $R_5$ Wasserstoff oder $C_{1-4}$-Alkyl bedeuten.

4. Derivate der Formel (I) nach Anspruch 1, dadurch gekennzeichnet, daß N $R_1$ $R_2$ einen Pyrrolidino-, Piperidino-, Homopiperidino- oder Morpholinoring bilden, der ggf. mit einem $C_{1-4}$-Alkyl substituiert ist.

5. Derivate der Formel (I) nach Anspruch 4, dadurch gekennzeichnet, daß N $R_1$ $R_2$ einen Morpholinoring, $R_3$ Wasserstoff oder $C_{1-4}$-Alkoxy und $R_4$ Wasserstoff oder $C_{1-4}$-Alkyl bedeuten.

6. Derivate der Formel (I) nach einem der Ansprüche 1 bis 5, dadurch gekennzeichnet, daß X sich in Stellung 6 des Chinolinrings befindet und Halogen, $C_{1-4}$-Alkyl oder Cycloalkyl, $C_{1-4}$-Alkoxy, Nitro oder Trifluormethyl bedeutet.

7. 2-[3-Ethoxycarbonyl-6-chlor-4-chinolyl]-amino-N,N-dipropylacetamid.

8. 2-N'-[3-Ethoxycarbonyl-6-chlor-4-chinolyl]-N'-methylamino-N,N-dipropylacetamid.

9. Verfahren zur Herstellung der Derivate der Formel (I) nach Anspruch 1, dadurch gekennzeichnet, daß ein Glycinderivat der Formel (II)

$$R_4\text{-HN-CH}_2\text{-CO-N}\begin{matrix}R_1\\R_2\end{matrix} \qquad (II)$$

mit einem 4-Chlorchinolin der Formel (III)

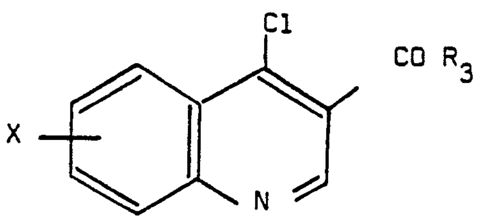

(III)

in denen $R_1$, $R_2$, $R_3$, $R_4$ und X die in Formel (I) in einem der Ansprüche 1 bis 6 angegebene Bedeutung haben, umgesetzt wird.

10. Verfahren nach Anspruch 9, dadurch gekennzeichnet, daß die Kondensation durch Erhitzen der beiden Bestandteile auf eine Temperatur von 60 bis 130°C durchgeführt wird.

11. Verfahren nach Anspruch 10, dadurch gekennzeichnet, daß die Kondensation in Gegenwart einer organischen oder anorganischen Base durchgeführt wird.

12. Verfahren nach Anspruch 10, dadurch gekennzeichnet, daß die Kondensation günstigerweise in einem organischen Lösungsmittel, wie Toluol, Ethyl- oder Isopropylalkohol, durchgeführt wird.

13. Arzneimittel, gekennzeichnet durch ein Derivat nach einem der Ansprüche 1 bis 8 als Wirkstoff.

14. Arzneimittel nach Anspruch 13, gekennzeichnet durch eine für die orale, parenterale oder rektale Verabreichung geeignete Form.

15. Arzneimittel nach Anspruch 13 oder 14, gekennzeichnet durch Einzeldosisformen mit einem Gehalt von 0,005 bis 0,300 g.

**Patentansprüche für den Vertragsstaat:** AT

1. Verfahren zur Herstellung von Chinolylglycinamid-Derivaten der folgenden allgemeinen Formel (I):

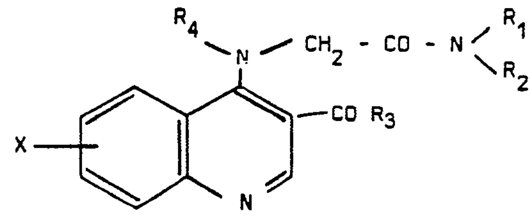

(I)

in der bedeuten:

$R_1$ und $R_2$, unabhängig voneinander, je Wasserstoff oder $C_{1-4}$-Alkyl, oder, wenn $R_1$ Alkyl ist, $R_2$ Cycloalkyl, ggf. mit einem Halogen oder einem $C_{1-4}$-Alkyl substituiertes Phenyl oder Benzyl, oder $R_1$ und $R_2$ zusammen mit dem Stickstoffatom, an das sie gebunden sind, einen Heterocyclus mit einem zweiten Heteroatom, wie Sauerstoff, Schwefel oder Stickstoff,

$R_3$ Wasserstoff, Hydroxyl, $C_{1-4}$-Alkyl oder $C_{1-4}$-Alkoxy, ggf. mit einem Halogen oder einem $C_{1-4}$-Alkyl substituiertes Phenoxy oder Benzyloxy oder -NH-$R_5$ mit $R_5$ Wasserstoff, $C_{1-4}$-Alkyl oder Phenyl,

$R_4$ Wasserstoff oder $C_{1-4}$-Alkyl,

X Wasserstoff oder Halogen, $C_{1-4}$-Alkyl, Cycloalkyl, Alkoxy, Nitro, Trifluormethyl oder Methylthio, sowie Ihre Säureadditionssalze mit pharmazeutisch verträglichen anorganischen oder organischen Säuren und ggf., wenn $R_3$ OH ist, Salzen mit pharmazeutisch verträglichen alkalischen oder organischen Basen, dadurch gekennzeichnet, daß ein Glycinderivat der Formel (II)

$$R_4\text{-HN-CH}_2\text{-CO-N}\begin{matrix}R_1\\R_2\end{matrix} \qquad (II)$$

mit einem 4-Chlorchinolin der Formel (III)

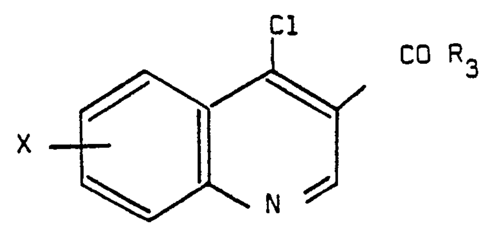

(III)

in der $R_1$, $R_2$, $R_3$, $R_4$ und X die in Formel (I) angegebene Bedeutung haben, umgesetzt wird.

2. Verfahren nach Anspruch 1, gekennzeichnet durch Verwendung von Verbindungen der Formeln (II) und (III), in denen $R_1$ und $R_2$ $C_{1-4}$-Alkyl, $R_3$ $C_{1-4}$-Alkoxy und $R_4$ Wasserstoff bedeuten.

3. Verfahren nach Anspruch 1, gekennzeichnet durch Verwendung einer Verbindung der Formeln (II), in der $R_1$, $R_2$ und $R_4$ $C_{1-4}$-Alkyl, $R_3$ $NHR_5$ mit $R_5$ Wasserstoff oder $C_{1-4}$-Alkyl bedeuten.

4. Verfahren nach Anspruch 1, gekennzeichnet durch Verwendung einer Verbindung der Formeln (II), in der N $R_1$ $R_2$ einen Pyrrolidino-, Piperidino-, Homopiperidino- oder Morpholinoring bilden, die ggf. mit einem $C_{1-4}$-Alkyl substituiert sind.

5. Verfahren nach Anspruch 4, gekennzeichnet

durch Verwendung einer Verbindung der Formeln (II), in der N $r_1$ $R_2$ einen Morpholinoring, $R_3$ Wasserstoff oder $C_{1-4}$-Alkoxy und $R_4$ Wasserstoff oder $C_{1-4}$-Alkyl bedeuten.

6. Verfahren nach einem der Ansprüche 1 bis 5, gekennzeichnet durch Verwendung einer Verbindung der Formel (III), in der X sich in Stellung 5 des Chinolinrings befindet und Halogen, $C_{1-4}$-Alkyl oder Cycloalkyl, $C_{1-4}$-Alkoxy, Nitro oder Trifluormethyl bedeutet.

7. Verfahren nach einem der Ansprüche 1 bis 6, dadurch gekennzeichnet, daß die Kondensation durch Erhitzen der beiden Bestandteile auf eine Temperatur von 60 bis 130°C durchgeführt wird.

8. Verfahren nach einem der Ansprüche 1 bis 7, dadurch gekennzeichnet, daß die Kondensation in Gegenwart einer organischen oder anorganischen Base durchgeführt wird.

9. Verfahren nach einem der Ansprüche 1 bis 8, dadurch gekennzeichnet, daß die Kondensation günstigerweise in einem organischen Lösungsmittel, wie Toluol, Ethyl- oder Isopropylalkohol, durchgeführt wird.

10. Verfahren zur Herstellung eines Arzneimittels, dadurch gekennzeichnet, daß das Arzneimittel ein nach einem der Ansprüche 1 bis 9 hergestelltes Derivat als Wirkstoff enthält.

**Claims for the contracting states:**
BE, CH, DE, FR, GB, IT, LI, LU, NL, SE

1. Quinolylglycine amide derivatives corresponding to the following general formula:

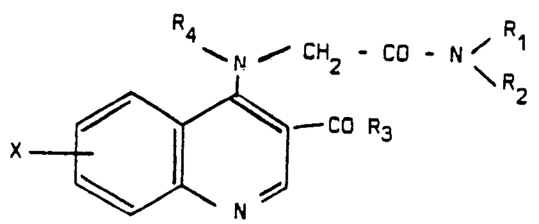
(I)

in which:

$R_1$ and $R_2$ each independently of one another represent a hydrogen or a $C_1$-$C_4$-alkyl radical, or if $R_1$ is an alkyl radical, $R_2$ can represent a cycloalkyl radical or a phenyl or benzyl radical, optionally substituted by a halogen or a $C_1$-$C_4$-alkyl, or $R_1$ and $R_2$ can form, with the nitrogen atom to which they are bonded, a heterocyclic radical which can contain a second hetero atom, such as oxygen, sulphur or nitrogen; $R_3$ represents a hydrogen, a hydroxyl OH, a $C_1$-$C_4$-alkyl or -alkoxy group or phenoxy or benzyloxy, optionally substituted by a halogen or a $C_1$-$C_4$-alkyl, or $R_3$ represents a grouping -NH-$R_5$, in which $R_5$ is a hydrogen, a $C_1$-$C_4$-alkyl radical or phenyl; $R_4$ represents a hydrogen or a $C_1$-$C_4$-alkyl radical; X represents a hydrogen atom or a halogen or a $C_1$-$C_4$-alkyl, cycloalkyl, alkoxy, nitro, trifluoromethyl or methylthio radical, and their addition salts with pharmaceutically acceptable inorganic or organic acids, and if appropriate, if $R_3$ represents OH, the salts with pharmaceutically acceptable alkali or organic bases.

2. Derivatives of the formula (I) according to claim 1, characterized in that $R_1$ and $R_2$ represent $C_1$-$C_4$-alkyl groups, $R_3$ represents a $C_1$-$C_4$-alkoxy group and $R_4$ represents a hydrogen.

3. Derivatives of the formula (I) according to claim 1, characterized in that $R_1$, $R_2$ and $R_4$ represent $C_1$-$C_4$-alkyl groups and $R_3$ represents an $NHR_5$ group, in which $R_5$ is hydrogen or a $C_1$-$C_4$-alkyl group.

4. Derivatives of the formula (I) according to claim 1, characterized in that $NR_1R_2$ forms a pyrrolidino, piperidino, homopiperidino or morpholino heterocyclic radical which can carry a $C_1$-$C_4$-alkyl substituent.

5. Derivatives of the formula (I) according to claim 4, characterized in that the group $NR_1R_2$ represents a morpholino group, $R_3$ represents hydrogen or a $C_1$-$C_4$-alkoxy group and $R_4$ represents hydrogen or a $C_1$-$C_4$-alkyl group.

6. Derivatives of the formula (I) according to one of claims 1 to 5, characterized in that X in the 6-position of the quinoline nucleus represents a halogen, a $C_1$-$C_4$-alkyl or cycloalkyl radical, $C_1$-$C_4$-alkoxy, nitro or trifluoromethyl.

7. 2-[4-(3-ethoxycarbonyl-6-chloro)-quinolyl]-amino-N,N-dipropylacetamide.

8. 2-[N'-[4-(3-ethoxycarbonyl-6-chloro)-quinolyl]-N'-methyl]amino-N,N-dipropylacetamide.

9. Process for the preparation of derivatives of the formula (I) according to claim 1, characterized in that a glycine derivative of the formula

$$R_4\text{-HN-CH}_2\text{-CO-N}\begin{matrix} R_1 \\ \\ R_2 \end{matrix} \quad \text{(II)}$$

is reacted with a 4-chloroquinoline of the formula

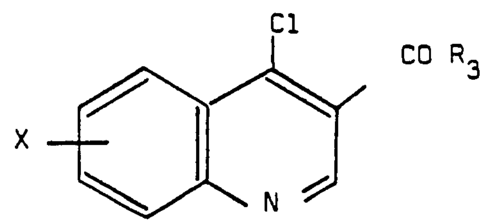
(III)

in which $R_1$, $R_2$, $R_3$, $R_4$ and X have the meanings indicated for the formula (I) in one of claims 1 to 6.

10. Process according to claim 9, characterized in that the condensation reaction is carried out by heating the 2 reagents at a temperature between 60°C and 130°C.

11. Process according to claim 10, characterized in that the condensation reaction is carried out in the presence of an organic or inorganic base.

12. Process according to claim 10, characterized in that the condensation reaction is advantageously carried out in an organic solvent, such as toluene or ethyl or isopropyl alcohol.

13. Medicament, characterized in that it contains a derivative according to one of claims 1 to 8 as the active principle.

14. Medicament according to claim 13, characterized in that it is presented in a form suitable for oral, parenteral or rectal administration.

15. Medicament according to claim 13 or 14, characterized in that it is presented in the form of unit doses containing 0.005 g to 0.300 g.

**Claims for the contracting state:** AT

1. Process for the preparation of quinolylglycine amide derivatives corresponding to the following general formula:

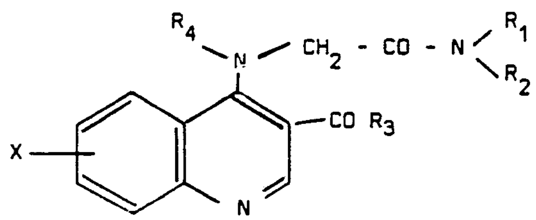

(I)

in which

$R_1$ and $R_2$ each independently of one another represent a hydrogen or a $C_1$-$C_4$-alkyl radical, or if $R_1$ is an alkyl radical, $R_2$ can represent a cycloalkyl radical or a phenyl or benzyl radical, optionally substituted by a halogen or a $C_1$-$C_4$-alkyl, or $R_1$ and $R_2$ can form, with the nitrogen atom to which they are bonded, a heterocyclic radical which can contain a second hetero atom, such as oxygen, sulphur or nitrogen; $R_3$ represents a hydrogen, a hydroxyl OH, a $C_1$-$C_4$-alkyl or -alkoxy group or phenoxy or benzyloxy, optionally substituted by a halogen or a $C_1$-$C_4$-alkyl, or $R_3$ represents a grouping -NH-$R_5$, in which $R_5$ is a hydrogen, a $C_1$-$C_4$-alkyl radical or phenyl; $R_4$ represents a hydrogen or a $C_1$-$C_4$-alkyl radical; X represents a hydrogen atom or a halogen or a $C_1$-$C_4$-alkyl, cycloalkyl, alkoxy, nitro, trifluoromethyl or methylthio radical, and their addition salts with pharmaceutically acceptable inorganic or organic acids, and if appropriate, if $R_3$ represents OH, the salts with pharmaceutically acceptable alkali or organic bases, characterized in that a glycine derivative of the formula

$$R_4\text{-HN-CH}_2\text{-CO-N}\langle^{R_1}_{R_2} \qquad \text{(II)}$$

is reacted with a 4-chloroquinoline of the formula

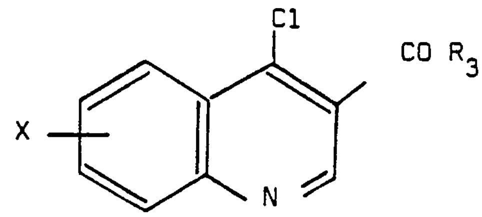

(III)

in which $R_1$, $R_2$, $R_3$, $R_4$ and X have the meanings indicated for the formula (I).

2. Process according to claim 1, characterized in that $R_1$ and $R_2$ represent $C_1$-$C_4$-alkyl groups, $R_3$ represents a $C_1$-$C_4$-alkoxy group and $R_4$ represents a hydrogen.

3. Process according to claim 1, characterized in that $R_1$, $R_2$ and $R_4$ represent $C_1$-$C_4$-alkyl groups and $R_3$ represents an $NHR_5$ group, in which $R_5$ is hydrogen or a $C_1$-$C_4$-alkyl group.

4. Process according to claim 1, characterized in that $NR_1R_2$ forms a pyrrolidino, piperidino, homopiperidino or morpholino heterocyclic radical which can carry a $C_1$-$C_4$-alkyl substituent.

5. Process according to claim 4, characterized in that the group $NR_1R_2$ represents a morpholino group, $R_3$ represents hydrogen or a $C_1$-$C_4$-alkoxy group and $R_4$ represents hydrogen or a $C_1$-$C_4$-alkyl group.

6. Process according to one of claims 1 to 5, characterized in that X in the 6-position of the quinoline nucleus represents a halogen, a $C_1$-$C_4$-alkyl or cycloalkyl radical, $C_1$-$C_4$-alkoxy, nitro or trifluoromethyl.

7. Process according to one of claims 1 to 6, characterized in that the condensation reaction is carried out by heating the 2 reagents at a temperature between 60°C and 130°C.

8. Process according to one of claims 1 to 7, characterized in that the condensation reaction is carried out in the presence of an organic or inorganic base.

9. Process according to one of claims 1 to 8, characterized in that the condensation reaction is advantageously carried out in an organic solvent, such as toluene or ethyl or isopropyl alcohol.

10. Process for the preparation of a medicament, characterized in that it contains a derivative obtained according to one of claims 1 to 9 as the active principle.